# FASCICULE DE BREVET EUROPEEN

(11) **EP 2 262 769 B1**
(45) Date de publication et mention de la délivrance du brevet: **05.11.2014**
(21) Numéro de dépôt: 09754044.7
(22) Date de dépôt: 20.03.2009
(51) Int. Cl.: C07D 213/28, C07D 233/56, C07D 471/04, A61K 31/437, A61P 19/00, A61P 25/00, A61P 35/00

(54) **DERIVES DE 2-ARYL-6-PHENYL-IMIDAZO[1,2-A]PYRIDINES POLYSUBSTITUES, LEUR PRÉPARATION ET LEUR APPLICATION EN THÉRAPEUTIQUE**
POLYSUBSTITUIERTE 2-ARYL-6-PHENYL-IMIDAZO-[1,2-A-]PYRIDIN-DERIVATE SOWIE DEREN HERSTELLUNG UND THERAPEUTISCHE ANWENDUNG
POLYSUBSTITUTED 2-ARYL-6-PHENYL-IMIDAZO[1,2-A]PYRIDINE DERIVATIVES, AND PREPARATION AND THERAPEUTIC USE THEREOF

(30) Priorité: 21.03.2008 FR 0801581
(43) Date de publication de la demande: 22.12.2010
(73) Titulaire: SANOFI, 75008 Paris (FR)
(72) Inventeur: ALMARIO GARCIA, Antonio, F-75013 Paris (FR); DE PERETTI, Danielle, F-75013 Paris (FR); EVANNO, Yannick, F-75013 Paris (FR); LARDENOIS, Patrick, F-75013 Paris (FR); MALANDA, André, F-75013 Paris (FR); RAKOTOARISOA, Nathalie, F-75013 Paris (FR)
(74) Mandataire: Le Coupanec, Pascale A.M.P.
(86) Numéro de dépôt international: PCT/FR2009/000302
(87) Numéro de publication internationale: WO 2009/144394

(56) Documents cités:
- WO-A-2008/034974
- FR-A- 2 903 105
- FR-A- 2 903 107

## Description

La présente invention se rapporte à des dérivés de 2-aryl-6-phényl-imidazo[1,2-a]pyridines polysubstitués, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

Les demandes WO2008/034974, FR2903105 et FR2903107 de la société déposante décrivent respectivement des dérivés.de 2-aryl-6-phényl-imidazo[1,2-α]pyridines, des dérivés de 2-benzoyl-imidazo[1,2-α]pyridines et des dérivés d'imidazo[1,2-α]pyridine-2-carboxamides, utiles pour la prévention ou le traitement de maladies impliquant les récepteurs nucléaires Nurr-1.

La présente invention décrit les composés de formule (I) : 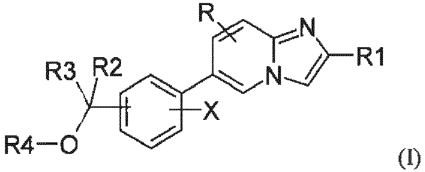 dans laquelle :
R₁ représente un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, halo(C₁-C₁₀)alkyle, (C₁-C₁₀)alcoxy, halo(C₁-C₁₀)alcoxy, (C₁-C₁₀)thioalkyle, - S(O)(C₁-C₁₀)alkyle, -S(O)₂(C₁-C₁₀-alkyle), hydroxyle, cyano, nitro, hydroxy(C₁-C₁₀)alkylène, NRaRb(C₁-C₁₀)alkylène, (C₁-C₁₀)alcoxy(C₁-C₁₀)alkylène-oxy, NRaRb, CONRaRb, SO₂NRaRb, NRcCORd, OC(O)NRaRb, OCO(C₁-C₁₀)alkyle, NRcC(O)ORe, NRcSO₂Rc, aryl(C₁-C₁₀)alkylène, aryle ou hétéroaryle monocyclique, l'aryle ou l'hétéroaryle monocyclique étant éventuellement substitués par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, oxo, nitro, cyano ou OCO(C₁-C₁₀)alkyle;
X représente de 1 à 4 substituants identiques ou différents l'un de l'autre choisis parmi hydrogène, halogène, (C₁-C₁₀)alkyle, (C₁-C₁₀)alcoxy, NRaRb, cyano, nitro, le (C₁-C₁₀)alkyle pouvant être éventuellement substitué par un ou plusieurs groupes choisis parmi un halogène, (C₁-C₁₀)alcoxy, halo(C₁-C₁₀) alcoxy, NRaRb, ou hydroxyle ;
R représente en position 3, 5, 7 ou 8 de l'imidazo[1,2-a]pyridine de 1 à 4 substituants identiques ou différents l'un de l'autre choisis parmi un halogène, (C₁-C₁₀)alkyle, halo(C₁-C₁₀)alkyle, (C₁-C₁₀)alcoxy ;
   R₂ et R₃ représentent, indépendamment l'un de l'autre,
   un atome d'hydrogène,
   un groupe (C₁-C₁₀)alkyle, éventuellement substitué par un groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy,
   hala(C₁-C₁₀)alcoxy, NRaRb, hydroxyle, nitro ou cyano ;
R₂ et X peuvent former ensemble avec les atomes de carbone qui les portent un cycle carboné de 5 à 7 atomes de carbone;
R₄ représente :
   un atome d'hydrogène,
   un groupe (C₁-C₁₀)alkyle, éventuellement substitué par un groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano, (C₁-C₁₀)alkyl(CO)-, CONRaRb, NRcCORd, OC(O)NRaRb, OCO(C₁-C₁₀)alkyle, NRcC(O)ORe ou aryle, l'aryle étant éventuellement substitué par un ou plusieurs substituant choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
Ra et Rb représentent, indépendamment l'un de l'autre, un atome d'hydrogène ou un groupe (C₁-C₁₀)alkyle, aryl(C₁-C₁₀)alkylène ou aryle ;
ou Ra et Rb forment ensemble, avec l'atome d'azote qui les porte, un groupe azétidine, pyrrolidine, pipéridine, épine, morpholine, thiomorpholine, pipérazine, homopipérazine, ce groupe étant éventuellement substitué par un groupe (C₁-C₁₀)alkyle, aryle ou aryl(C₁-C₁₀)alkylène ; Rc et Rd représentent, indépendamment l'un de l'autre, un atome d'hydrogène ou un groupe (C₁-C₁₀)alkyle, aryl(C₁-C₁₀)alkylène, aryle,
ou Rc et Rd forment ensemble un groupe (C₂-C₃)alkylène ;
   Re représente un groupe (C₁-C₁₀)alkyle, aryl(C₁-C₁₀)alkylène, aryle,
ou Rc et Re forment ensemble un groupe (C₂-C₅)alkylène ;
   Rf représente un d'halogène, un groupe (C₁-C₁₀)akoxy, halo(C₁-C₁₀)alcoxy, hydroxyle, cyano, NRaRb, C(O)NRaRb, NRcCORd, OC(O)NRaRb, OCO(C₁-C₁₀)alkyle, NRcCOORe, SO₂NRaRb, NRcSO₂Re, aryl(C₁-C₁₀)alkylène, aryle, l'aryle étant éventuellement substitué par un ou plusieurs substituants choisi parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano ou OCO(C₁-C₁₀)alkyle; à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl, cyclopropylméthyl etc ;
- un groupe alkylène : un groupe alkyle divalent
- un groupe alcoxy : un radical -O-alkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃, CHF₂, CCl₃.
- un groupe haloalcoxy : un radical -O-alkyle où le groupe alkyle est tel que précédemment défini et substitué par un ou plusieurs atomes d'halogène identiques ou différents. A titre d'exemples, on peut citer les groupes OCF₃, OCHF₂, OCCl₃.
- un groupe thioalkyle : un radical S-alkyle où le groupe alkyle est tel que précédemment défini ;
- un groupe aryle : un groupe aromatique mono ou bicyclique comportant de 6 à 10 atomes. A titre d'exemples de groupes aryles, on peut citer phényle et naphthyle ;
- un groupe hétéroaryle : un groupe aromatique mono ou bicyclique comportant de 5 à 10 atomes dont de 1 à 4 hétéroatomes choisis parmi N, O et S. A titre d'exemples de groupes hétéroaryles monocycliques, on peut citer : pyrrole, furane, thiophène, pyrazole, imidazole, thiazole, tetrazole, oxazole, isoxazole, oxadiazole, thiazole, isothiazole, thiadiazole, pyridine, pyrimidine, pyrazine, pyridazine, triazine ;
- les atomes de soufre et d'azote peuvent être à l'état oxydé (N-oxide, sulfoxide, sulfone).

Parmi les composés de formule (I), figurent les composés (I) objets de l'invention pour lesquels:
R₁ représente un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, (C₁-C₁₀)alcoxy, halo(C₁-C₁₀)alcoxy,
X représente de 1 à 2 substituants identiques ou différents l'un de l'autre choisis parmi hydrogène, halogène ;
R représente en position 3, 7 ou 8 de l'imidazo[1,2-α]pyridine de 1 à 2 substituants identiques ou différents l'un de l'autre choisis parmi un halogène, (C₁-C₁₀)alkyle ;
R₂ et R₃ représentent, indépendamment l'un de l'autre,
un atome d'hydrogène,
un groupe (C₁-C₁₀)alkyle;
R₄ représente :
   un atome d'hydrogène,
   un groupe (C₁-C₁₀)alkyle;
à l'état de base ou de sel d'addition à un acide.

Parmi les composés de formule (I), un premier groupe de composés est constitué des composés pour lesquels :
R₁ représente un groupe phényle ou un groupe naphtyle, éventuellement substitué par un ou plusieurs atomes ou groupes choisis indépendamment les uns des autres parmi les atomes d'halogène ou groupes (C₁-C₁₀)alkyle, (C₁-C₁₀)alcoxy, halo(C₁-C₁₀)alcoxy ;
les autres substituant étant tel que définis précédemment.

Parmi les composés de formule (I), un deuxième groupe de composés est constitué des composés pour lesquels :
R₁ représente un groupe phényle ou un groupe naphtyle, le groupe phényle étant éventuellement substitué en position 2, 3 ou 4 par un ou plusieurs atomes ou groupes choisis indépendamment les uns des autres parmi les atomes d'halogène ou groupes (C₁-C₁₀)alkyle, (C₁-C₁₀)alcoxy, halo(C₁-C₁₀)alcoxy ;
les autres substituant étant tel que définis précédemment.

Parmi les composés de formule (I), un troisième groupe de composés est constitué des composés pour lesquels :
R₁ représente un groupe phényle ou un groupe naphtyle, éventuellement substitué par un ou plusieurs atomes ou groupes choisis indépendamment les uns des autres parmi les atomes d'halogène ou groupes méthoxy, méthyle, difluorométhyloxy ;
les autres substituant étant tel que définis précédemment.

Parmi les composés de formule (I), un quatrième groupe de composés est constitué des composés pour lesquels :
R₁ représente un groupe phényle un groupe naphtyle, le groupe phényle étant éventuellement substitué en position 2, 3 ou 4 par un ou plusieurs atomes ou groupes choisis indépendamment les uns des autres parmi les atomes d'halogène ou groupes méthoxy, éthyle, difluorométhyloxy ;
les autres substituant étant tel que définis précédemment.

Parmi les composés de formule (I), un cinquième groupe de composés est constitué des composés pour lesquels :
X représente 1 ou 2 substituant identiques ou différents l'un de l'autre choisis parmi les atomes d'hydrogène, d'halogène ;
les autres substituant étant tel que définis précédemment.

Parmi les composés de formule (I), un sixième groupe de composés est constitué des composés pour lesquels :
X représente 1 ou 2 substituant identiques ou différents l'un de l'autre choisis parmi les atomes d'hydrogène, de fluor ;
les autres substituant étant tel que définis précédemment.

Parmi les composés de formule (I), un septième groupe de composés est constitué des composés pour lesquels :
R représente, en position 3, 7 ou 8 de l'imidazo[1,2-a]pyridine, 1 ou 2 substituant identiques ou différents l'un de l'autre choisis parmi les atomes d'halogène, les groupes (C₁-C₁₀)alkyle ;
les autres substituant étant tels que définis précédemment.

Parmi les composés de formule (I), un huitième groupe de composés est constitué des composés pour lesquels :
R représente, en position 3, 7 ou 8 de l'Imidazo[1,2-a]pyridine, un substituant choisi parmi les atomes d'halogène, les groupes (C₁-C₁₀)alkyle ;
les autres substituant étant tels que définis précédemment.

Parmi les composés de formule (I), un neuvième groupe de composés est constitué des composés pour lesquels :
R représente, en position 3, 7 ou 8 de l'imidazo[1,2-a]pyridine, un substituant choisi parmi les atomes d'halogène, le groupe méthyle ;
les autres substituant étant tels que définis précédemment.

Parmi les composés de formule (I), un dixième groupe de composés est constitué des composés pour lesquels :
R₂ et R₃ représentent indépendamment l'un de l'autre un un atome d'hydrogène ou un groupe (C1-C₁₀)alkyle ;
les autres substituant étant tels que définis précédemment.

Parmi les composés de formule (I), un onzième groupe de composés est constitué des composés pour lesquels :
R₂ et R₃ représentent indépendamment l'un de l'autre un un atome d'hydrogène ou un groupe méthyle ;
les autres substituant étant tels que définis précédemment.

Parmi les composés de formule (I), un douzième groupe de composés est constitué des composés pour lesquels :
R₄ représente un atome d'hydrogène ou un groupe (C₁-C₁₀)alkyle ;
les autres substituant étant tels que définis précédemment.

Parmi les composés de formule (I), un treizième groupe de composés est constitué des composés pour lesquels :
R₄ représente un atome d'hydrogène ou un groupe méthyle ;
les autres substituants étant tels que définis précédemment.

Parmi les composés de formule (I) objets de l'invention, un quatorzième groupe de composés est constitué des composés pour lesquels : Le groupe 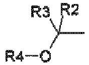 est en position 2, 3 ou 4 sur le noyau phényle qui le porte ; les autres substituants des composés de formule (I) étant définis tels que précédemment.

Parmi les composés de formule (I), un quinzième groupe de composés est constitué des composés pour lesquels :
R₁ représente un groupe phényle ou un groupe naphtyle, éventuellement substitué par un ou
plusieurs atomes ou groupes choisis indépendamment les uns des autres parmi les atomes d'halogène ou groupes (C₁-C₁₀)alkyle, (C₁-C₁₀)alcoxy, halo(C₁-C₁₀)alcoxy ;
X représente 1 ou 2 substituants identiques ou différents l'un de l'autre choisis parmi les atomes d'hydrogène, d'halogéne ;
R représente, en position 3, 7 ou 8 de l'imidazo[1,2-a]pyridine, un substituant choisi parmi les atomes halogène, les groupes (C₁-C₁₀)alkyle ;
R₂ et R₃ représentent indépendamment l'un de l'autre un un atome d'hydrogène ou un groupe (C₁-C₁₀)alkyle ;
représente un atome d'hydrogène ou un groupe (C₁-C₁₀)alkyle ;

Le groupe 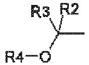 est en position 2, 3 ou 4 sur le noyau phényle qui le porte ; à l'état de base ou de sel d'addition à un acide.

Parmi les composés de formule (I), un seizième groupe de composés est constitué des composés pour lesquels :
R₁ représente un groupe phényle un groupe naphtyle, le groupe phényle étant éventuellement substitué en position 2, 3 ou 4 par un ou plusieurs atomes ou groupes choisis indépendamment les uns des autres parmi les atomes d'halogène ou groupes méthoxy, méthyle, difluorométhyloxy ;
X représente 1 ou 2 substituants identiques ou différents l'un de l'autre choisis parmi les atomes d'hydrogène, de fluor ;
R représente, en position 3, 7 ou 8 de l'imidazo[1,2-a]pyridine, un substituant choisi parmi les halogènes, le groupe méthyle ;
R₂ et R₃ représentent indépendamment l'un de l'autre un atome d'hydrogène ou un groupe méthyle ;
R₄ représente un atome d'hydrogène ou un groupe méthyle ;

Le groupe 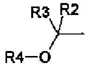 est en position 2, 3 ou 4 sur le noyau phényle qui le porte ; à l'état de base ou de sel d'addition à un acide.

Parmi les composés de formule (I) objets de l'invention, un dix-septième groupe de composés est constitué des composés pour lesquels :
R₁ représente un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, (C₁-C₁₀)alcoxy, hato(C₁-C₁₀)alcoxy ;
X représente un atome d'hydrogène ;
R est en position 3, 7 ou 8 de l'imidazo[1,2-*a*]pyridine et représente un (C₁-C₁₀)alkyle ;
R₂ et R₃ représentent, indépendamment l'un de l'autre, un atome d'hydrogène ;
R₄ représente un atome d'hydrogène ;
à l'état de base ou de sel d'addition à un acide.

Parmi les composés de formule (I) objets de l'invention, on peut notamment citer les composés suivants :
{3-[2-(4-Chlorophényl)-8-méthylimidazo[1,2-*a*]pyridin-6-yl]phényl}méthanol ;
{3-[2-(4-Chlorophényl)-7-méthylimidazo[1,2-*a*]pyridin-6-yl]phényl}méthanol ;
[3-((3-Méthyl-2-phénylimidazo[1,2-*a*]pyridin-6-yl) phényl]méthanol ;
{3-[2-(2,4-difluorophényl)-3-méthylimidazo[1,2-*a*]pyridin-6-yl]phényl}méthanol et son chlorhydrate ;
{3-[2-(4-chloro-3-éthylphényl)-3-méthylimidazo[1,2-*a*]pyridin-6-yl]phényl}méthanol et son chlorhydrate ;
{3-[2-(4-chlorophényl)-3-méthylimidazo[1,2-*a*]pyridin-6-yl]phényl}méthanol et son chlorhydrate ;
{3-[2-(4-méthoxyphényl)-3-méthylimidazo[1,2-*a*]pyridin-6-yl]phényl}méthanol et son chlorhydrate ;
{3-[2-[4-(difluorométhyloxy)phényl]-3-méthylimidazo[1,2-*a*]pyridin-6-yl]phényl}méthanol et son chlorhydrate ;
[3-((8-Méthyl-2-naphthyl-2-ylimidazo[1,2-*a*]pyridin-6-yl)phényl]méthanol ;
[4-((8-Méthyl-2-naphthyl-2-ylimidazo[1,2-*a*]pyridin-6-yl)phényl]méthanol ;
2-[3-(3-Méthyl-2-phénylimidazo[1,2-*a*]pyridin-6-yl)phényl]propan-2-ol ;
2-{3-[2-(4-Chlorophényl)-3-méthylimidazo[1,2-*a*]pyridin-6-yl]phényl}propan-2-ol ;
2-{3-[2-{3-Chlorophényl)-3-méthylimidazo[1,2-*a*]pyridin-6-yl]phényl}propan-2-ol ;
2-{3-[2-(2,4-Difluorophényl)-3-méthylimidazo[1,2-*a*]pyridin-6-yl]phényl]propan-2-ol ;
2-{3-[2-(4-Méthoxyphényl)-3-méthylimidazo[1,2-*a*]pyridin-6-yl]phényl}propan-2-ol;
{2-Fluoro-6-[2-(4-méthoxyphényl)-3-méthylimidazo[1,2-*a*]pyridin-6-yl]phényl}méthanol ;
[2,6-Difluoro-3-(3-méthyl-2-phénylimidazo[1,2-*a*]pyridin-6-yl)phényl]méthanol ;
{3-[2-(4-Chlorophényl)-3-méthylimidazo[1,2-*a*]pyridin-6-yl]-2,6-difluorophényl}méthanol ;
{3-[2-(3-Chlorophényl)-3-méthylimidazo[1,2-*a*]pyridin-6-yl]-2,6-difluorophényl}méthanol ;
{2,6-Difluoro-3-[2-(4-méthoxyphényl)-3-méthylimidazo[1,2-*a*]pyridin-6-yl]-phényl}méthanol ;
{3-[3-Chloro-2-(4-chlorophénylimidazo[1,2-*a*]pyridin-6-yl]phényl}méthanol
2-{3-[2-(4-Chlorophényl)-3-fluoroimidazo[1,2-*a*]pyridin-6-yl]phényl}propan-2-ol ;
2-{3-[3-Chloro-2-(4-chlorophényl)imidazo[1,2-*a*]pyridin-6-yl]phényl}propan-2-ol;
[2-Fluoro-6-(3-méthyl-2-phénylimidazo[1,2-*a*]pyridin-6-yl)phényl]méthanol ;
{2-[2-(4-Chlorophényl)-3-méthylimidazo[1,2-*a*]pyridin-6-yl]-6-fluorophényl}méthanol ;
{2-[2-(3-Chlorophényl)-3-methylimidazo[1,2-*a*]pyridin-6-yl]-6-fluorophényl}méthanol ;
{2-[2-(2,4-Difluorophényl)-3-méthylimidazo[1,2-*a*]pyridin-6-yl]-6-fluorophényl}méthanol ;
3-Chloro-6-(3-méthoxyméthylphényl)-2-(4-chlorophényl)imidazo[1,2-*a*]pyridine;
{3-[3-Chloro-2-(4-chlorophényl)imidazo[1,2-*a*]pyridin-6-yl]-2,6-difluorophényl}méthanol.

Conformément à l'invention, on peut préparer les composés de formule générale (I) selon le procédé décrit dans le schéma 1.

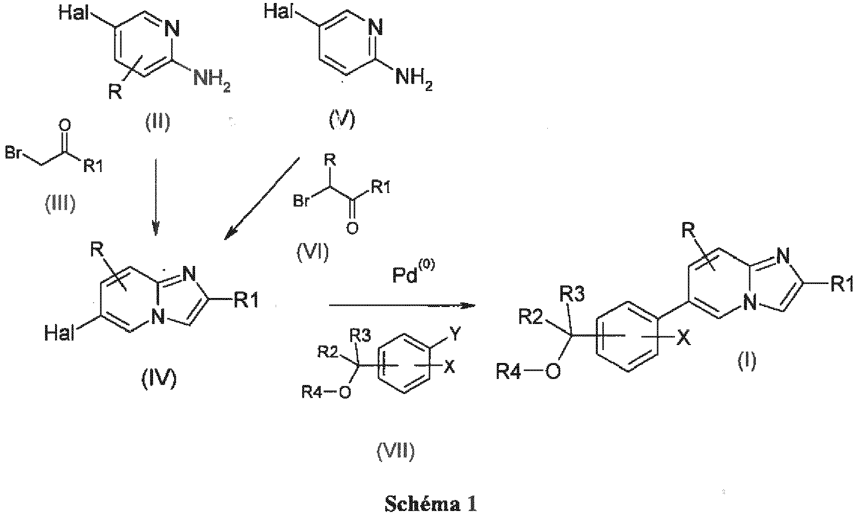

On peut préparer les composés de l'invention suivant le schéma 1 par une réaction de couplage, catalysée par un métal tel que le palladium, entre une imidazopyridine de formule générale (IV), dans laquelle R et R1 sont définis comme précédemment et Hal représente un atome d'halogène, et un dérivé de formule générale (VII), dans laquelle R2, R3, R4 et X sont définis comme précédemment et Y représente un dérivé de bore ou d'étain, pour obtenir les composés de formule générale (I), par exemple selon la méthode décrite par A. Gueiffier dans Helv. Chim. Acta 2001, 84, 3610-3615.

Les composés de formule générale (IV) peuvent être obtenus par condensation entre une aminopyridine de formule générale (II), dans laquelle R est défini comme précédemment et Hal représente un atome d'halogène, et une bromocétone de formule générale (III), dans laquelle R1 est défini comme précédemment, par exemple selon les méthodes décrites par L. Cai dans J. Med. Chem. 2007, 50, 4746, pour obtenir les composés de formule générale (IV), pour lesquels R est en position 5, 7 ou 8 du noyau imidazopyridine. Les composés de formule générale (IV), pour lesquels R est en position 3 du noyau imidazopyridine peuvent être obtenus par condensation entre une aminopyridine de formule générale (V), dans laquelle Hal représente un atome d'halogène, et une bromocétone de formule générale (VI), dans laquelle R et R1 sont définis comme précédemment, par exemple selon la méthode décrite par M. Fisher dans J. Med. Chem 1972, 15, 982.

Conformément à l'invention, on peut préparer les composés de formule générale (I), dans laquelle R est en position 3 du noyau imidazopyridine et représente un groupe alkyle, selon le procédé décrit dans le schéma 2.

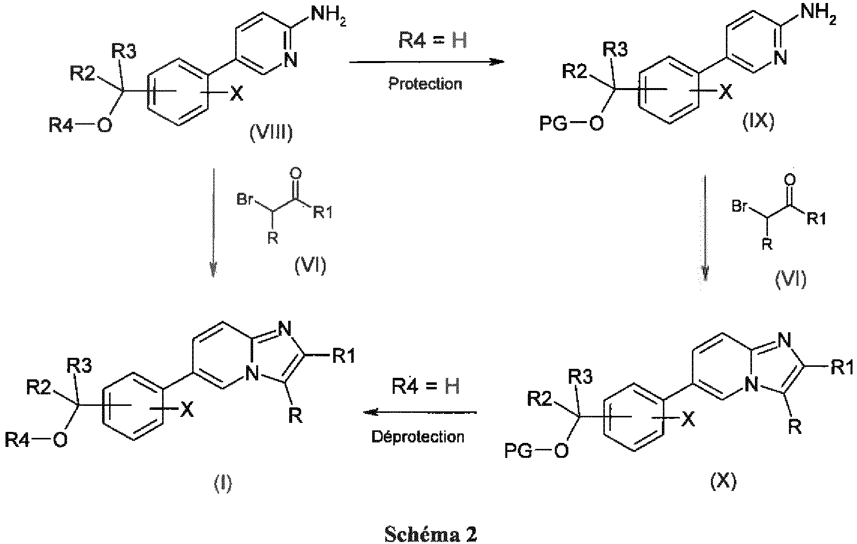

On peut préparer les composés de l'invention dans lesquels R est en position 3 du noyau imidazopyridine suivant le schéma 2 par une réaction de condensation entre une aminopyridine de formule générale (VIII), dans laquelle R2, R3 et R4 et X sont définis comme précédemment, R4 n'étant pas un atome d'hydrogène, et une bromocétone de formule générale (VI), dans laquelle R1 est défini comme précédemment et R représente un groupe alkyle, par exemple selon la méthode décrite par M. Fisher dans J. Med. Chem 1972, 15, 982.

Lorsque R4 représente un atome d'hydrogène, les composés de formule générale (VIII) peuvent être transformés en composés de formule générale (IX), dans laquelle R2, R3 et X sont définis tels que précédemment, et PG représente un groupement protecteur de fonction hydroxyle, tel que décrit par exemple par T. Greene dans « Protective Groups in Organic Synthesis » (Wiley Interscience*),* par exemple un groupe terbutyldiméthylsilyl, par toute méthode connue de l'Homme du métier. Les composés de formule générale (VIII) sont soumis à une réaction de condensation avec des bromocétones de formule générale (VI), dans laquelle R et R1 sont définis comme précédemment, pour obtenir des composés de formule générale (X), dans laquelle, R, R1, R2, R3 et X sont définis comme précédemment et PG représente un groupement protecteur d'hydroxyle. Enfin, les composés de formule générale (IX) sont soumis à une réaction de déprotection, comme décrit par exemple par T. Greene dans « Projective Groups in Organic Synthesis » (Wiley Interscience), pour obtenir les composés de formule générale (I), dans laquelle R est en position 3 du noyau imidazopyridine et R4 représente l'atome d'hydrogène.

Dans le schéma 2, les composés de départ et les réactifs, quand leur mode de préparation n'est pas décrit, sont disponibles dans le commerce ou décrits dans la littérature (par exemple WO2004076412), ou bien peuvent être préparés selon des méthodes qui y sont décrites ou qui sont connues de l'Homme du métier. En particulier, les aminopyridines de formule générale (VRIII) peuvent être obtenues selon des méthodes connues de l'homme du métier.

Conformément à l'invention, on peut préparer les composés de formule générale (I), dans laquelle R est en position 3 du noyau imidazopyridine et représente un atome d'halogène, selon le procédé décrit dans le schéma 3

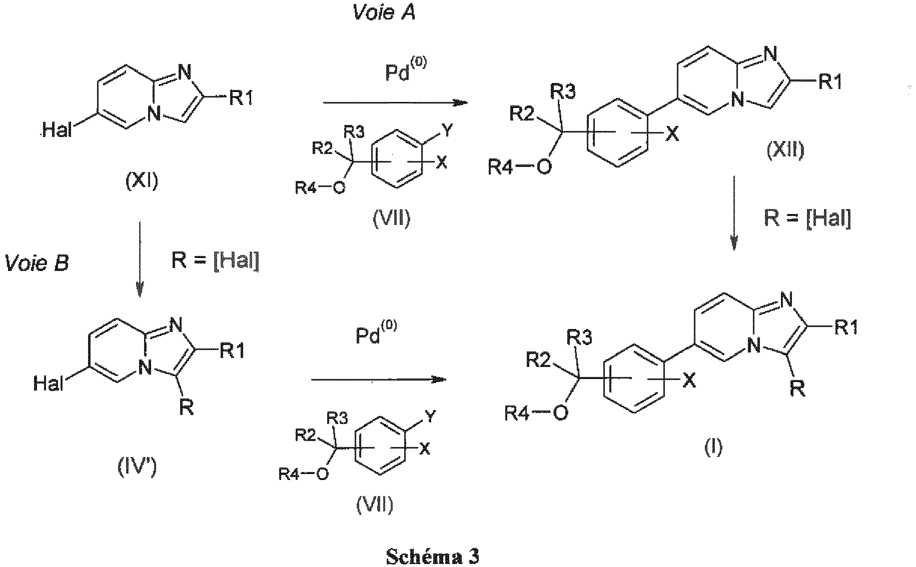

On peut préparer les composés de l'invention de formule générale (I) dans laquelle X, R1, R2, R3, R4 sont définis comme précédemment et R est en position 3 du noyau imidazopyridine et représente un halogène suivant le schéma 3 *voie A* par une réaction d'halogénation d'une imidazopyridine de formule générale (XII) dans laquelle X, R1, R2, R3, R4 sont définis comme précédemment au moyen d'un agent d'halogénation tel que le *N-*chlorosuccinimide. ou d'un agent de fluoration tel que le 1-chlorométhyl-4-fluoro-1,4-diazoniabicyclo[2.2.2]octane bis-tétrafluoroborate, connu également sous la dénomination commerciale « selectfluor^{®} ». Les composés de formule générale (XII) peuvent être obtenus par une réaction de couplage, catalysée par un métal tel que le palladium, entre une imidazopyridine de formule générale (XI), dans laquelle R1 est défini comme précédemment et Hal représente un atome d'halogène, et un dérivé de formule générale (VII), dans laquelle R2, R3, R4 et X sont définis comme précédemment et Y représente un dérivé de bore ou d'étain, par exemple selon la méthode décrite par A. Gueiffier dans Helv. Chim. Acta 2001, 84, 3610-3615.

Alternativement, on peut préparer les composés de l'invention de formule générale (I) dans laquelle X, R1, R2, R3, R4 sont définis comme précédemment et R est en position 3 du noyau imidazopyridine et représente un halogène suivant le schéma 3 *voie B* par une réaction de couplage catalysée par un métal tel que le palladium entre une imidazopyridine de formule générale (IV'), dans laquelle R1 est défini comme précédemment, R représente un atome de fluor ou de chlore et Hal représente un atome de brome ou d'iode, et un dérivé de formule générale (VII), dans laquelle R2, R3, R4 et X sont définis comme précédemment et Y représente un dérivé de bore ou d'étain, pour obtenir les composés de formule générale (I), par exemple selon la méthode décrite par A. Gueifier dans Helv. Chim. Acta 2001, 84, 3610-3615. Les composés de formule générale (IV') peuvent être obtenus par halogénation des imidazopyridines de formule (XI) au moyen d'un agent tel que le N-chlorosuccinimide.

Les produits de formule (I), peuvent être soumis, si désiré et si nécessaire, à toutes réactions connues de l'homme de l'art, dans un ordre quelconque, pour être transformés en d'autres produits de formule (I).

A titre d'exemples de réactions, on peut citer : les réactions d'estérifcation ou d'amidification de fonction acide, les réactions de carbamoylation, les réactions d'hydrolyse de fonction ester, les réactions de transformation de fonction hydroxyle en fonction alcoxy, les réactions de couplage catalysées par un métal de transition, les réactions de protection des fonctions réactives, les réaction d'élimination des groupements protecteurs que peuvent porter les fonctions réactives protégées, les réactions de salification par un acide minéral ou organique ou par une base pour obtenir le sel correspondant, les réactions de dédoublement des formes racémiques en énantiomères, lesdits produits de formule (I) ainsi obtenus étant le cas échéant sous toutes les formes isomères possibles racémiques, énantiomères et diastéréoisomères.

L'invention, selon un autre de ses aspects, a également pour objet les composés de formules (VIIIa), (VIIIb), (IXa), (IXb), (IXc) et (IV'a). Ces composés sont utiles comme intermédiaires de synthèse des composés de formule (I).

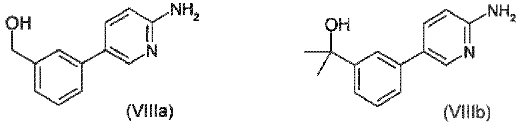

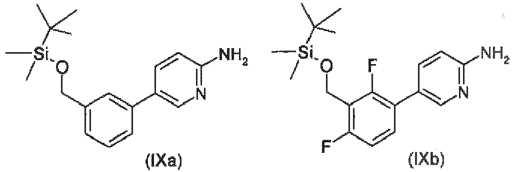

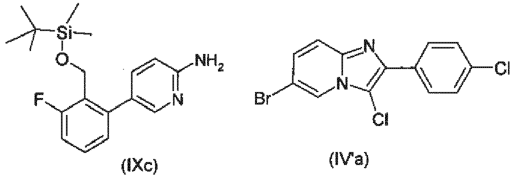

Le composé de formule (VIIIa) peut être préparé par une réaction de couplage catalysée par un métal, tel que le palladium, entre la 5-bromo-2-aminopyridine et un dérivé d'acide boronique, par exemple l'acide 3-(hydroxyméthyl)phénylboronique, tel que décrit dans l'exemple N°3. Le composé de formule (VIIIb) peut être préparé par une réaction de couplage catalysée par un métal, tel que le palladium, entre le 2-(3-bromophényl)propan-2-ol et une aminopyridine substituée par un dérivé de bore telle que, par exemple, une 5-(4,4,5,5-tétraméthyl-1,3,2-dioxaborolan-2-yl)pyridin-2-ylamine, tel que décrit dans l'exemple N°4. Le composé de formule (IXa) peut être préparé par action du (*tert-*butylchlorodiméthyl}silane sur le composé (VIIIa) dans un solvant, par exemple le tétrahydrofurane, en présence d'une base telle que l'imidazole, tel que décrit dans l'exemple N°3. Les composés de formule (IXb) et (IXc) peuvent être obtenus par une réaction de couplage catalysée par un métal tel que le palladium entre une aminopyridine substituée par un dérivé de bore telle que, par exemple, une 5-(4,4,5,5-tétraméthyl-1,3,2-dioxaborolan-2-yl)pyridin-2-ylamine et un dérivé de (tert-butyldiméthyloxy)méthylbromobenzène, tel que décrit dans l'exemple N°5. Le composé de formule (IV'a) peut être obtenu par action d'un agent d'halogénation tel que la N-chlorosuccinimide sur la 6-bromo-2-(4-chlorophényl)imidazo[1,2-a]pyridine tel que décrit dans l'exemple N°6.

Les composés de formules (VIIIa), (VIIIb), (IXa), (IXb), (IXc), (IV'a) ont été préparés à l'état de poudre ou d'huile, à l'état de base. Le tableau 1 rassemble quelques données physicochimiques de ces intermédiaires.

**Tableau 1**

| N° | R.M.N. ¹H (DMSO-d6, δ ppm) ; M+H ; PF |
|---|---|
| (VIIIa) | 4,55 (d, 2H) ; 5,2 (t, 1H) ; 6,05 (s, 2H) ; 6,55 (d,1H) ; 7,2 (d, 1H) ; de 7,3 à 7,55 (m, 3H) ; 7,7 (d, 1H) ; 8,25 (s, 1H) ; M+H = 201 |
| (VIIIb) | 1,45 (s, 6H) ; 5,0 (s, 1H) ; 6,0 (s, 2H) 6,55 (d, 1H) ; de 7,3 à 7,4 (m, 3H) ; 7,65 (s, 1H) ; 7,7 (d, 1H) ; 8,25 (s, 1H). M+H = 229 |
| (IXa) | 0,09 (s, 6H) ; 0,91 (s, 9H) ; 4,75 (s, 2H) ; 6,02 (m, 2H) ; 6,52 (dd, 1H) ; 7,21 (d, 1H) ; 7,36 (t, 1H) ; 7,43 (d, 1H) ; 7,47 (s, 1H) ; 7,66 (dd, 1H) ; 8,21 (d, 1H). M+H = 315 ; PF = 82 - 84°C |
| (IXb) | 0 (s, 6H) ; 0,8 (s, 9H) ; 4,4 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351 |
| (IXc) | 0 (s, 6H) ; 0,85 (s, 9H) ; 4,5 (s, 2H) ; 6,05 (s, 2H) ; 6,45 (d, 1H) ; de 7,05 à 7,15 (m, 2H) ; de 7,3 à 7,4 (m, 1H) ; de 7,45 à 7,5 (m, 1H) ; 8,0 (d, 1H). M+H = 333 |
| (IV'a) | 7,55 (d, 1H) ; 7,6 (d, 2H) ; 7,7 (d, 1H) ; 8,15 (d, 2H) 8,7 (s, 1H) |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 2 ci-après, qui illustre les structures chimiques de quelques composés selon l'invention.

La nomenclature des composés a été établie à partir du logiciel Autonom.

### Exemple 1 : {3-[2-(4-Chlorophényl)-8-méthylimidazo[1,2-a]pyridin-6-yl]phényl} éthanol (composé 1 du tableau)

### 1.1 6-Bromo-2-(4-chlorophényl)-8-méthylimidazo[1,2-a]pyridine

On place dans un ballon 1,70 g de 2-amino-5-bromo-3-méthylpyridine, 2,13 g de 2-bromo-1-(4-chlorophényl)éthanone dans 75 ml de n-propanol. On ajoute 1,08 g d'hydrogénocarbonate de sodium. On chauffe à 80°C pendant 6h. On laisse refroidir et le solvant est évaporé sous pression réduite. Le résidu est repris entre le dichlorométhane et l'eau, la phase organique est ensuite séparée, séchée et le filtrat est concentré sous pression réduite. Le résidu est purifié par chromatographie sur gel de silice en éluant avec un mélange dichlorométhane/méthanol. Le solide obtenu est trituré dans de l'éther diéthylique et recueilli par filtration. On obtient 1,7 g de composé.
RMN 1H (CDCl₃-d3, δ en ppm) : 2,6 (s, 3H) ; 7,0 (s, 1H) ; 7,4 (d, 2H) ; 7,7 (s, 1H) ; 7,9 (d, 2H) ; 8,1 (s, 1H).M+H=322.

### 1.2 {3-[2-(4-Chlorophényl)-8-méthylimidazo[1,2-a]pyridin-6-yl]phényl}méthanol

On place dans un ballon 10,5 ml d'acétonitrile, 10,5 ml de toluène et 8,5 ml d'une solution 2M de carbonate de sodium, on dégaze à l'argon pendant 10 min. On ajoute 704 mg de 6-bromo-2-(4-chlorophényl)-8-méthylimidazo[1,2-*a*]pyridine, 497 mg d'acide 3-hydroxyméthylphénylboronique et 126 mg de tétrakis(triphénylphosphine) palladium. On agite le mélange 5 h à 75°C. Après refroidissement, on élimine par filtration le catalyseur que l'on lave à l'acétate d'éthyle. La phase organique est séparée, séchée et le filtrat est concentré sous pression réduite. Le résidu est purifié par chromatographie sur gel de silice en éluant par un mélange dichlorométhane/méthanol. Le solide obtenu est trituré à l'éther diéthylique, recueilli par filtration puis séché à l'étuve sous pression réduite. On obtient 431 mg de composé.
PF = 174-177°C. RMN 1H (DMSO-d6, δ en ppm) : 2,6 (s, 3H) ; 4,6 (d, 2H) ; 5,3 (t, 1H) ; 7,3 (d, 1H) ; de 7,4 à 7,5 (m, 4H) ; 7,6 (d, 1H) ; 7,7 (s, 1H) ; 8,0 (d, 2H) ; 8,4 (s, 1H) ; 8,7 (s, 1H). M+H = 349.

### Exemple 2 : [3-(3-Méthyl-2-phénylimidazo[1,2-a]pyridin-6-yl)phényl]methanol (composé 3 du tableau)

### 2.1 6-Bromo-3-méthyl-2-phénylimidazo[1,2-a]pyridine

Dans un ballon de 100 ml, on dissout 865 mg de 2-amino-5-bromopyridine dans 25 ml d'acétone, puis ajoute 1,06 g de 2-bromo-1-phényl-1-propanone et chauffe le mélange au reflux du solvant pendant 57 heures. On laisse refroidir le mélange réactionnel puis recueille le précipité par filtration, le lave à l'éther diéthylique et le sèche sous pression réduite. On obtient 350 mg de composé.
RMN 1H (DMSO-d6, δ en ppm) : 2,82 (s, 3) ; de 7,55 à 7,9 (m, 5H) ; 7,95 (d, 1H, J = 4,2 Hz) ; 8,1 (d, 1H, J = 4,2Hz) ; 9,22 (s, 1H). M+H = 287.

### 2.2 [3-(3-Méthyl-2-phénylimidazo[1,2-a]pyridin-6-yl)phényl]méthanol

On place dans un ballon 5 ml d'acétonitrile, 5 ml de toluène et 5 ml d'une solution 2M de carbonate de sodium, on dégaze à l'argon pendant 15 min puis on ajoute 355 mg de 6-bromo-3-méthyl-2-phénylimidazo[1,2-*a*]pyridine, 282 mg d'acide 3-hydroxyméthylphénylboronique et 71 mg de tétrakis(triphénylphosphine)palladium. On agite le mélange 16 h dans un bain thermostaté à 90°C. Le mélange réactionnel est ensuite refroidi à température ambiante et les solvants sont évaporés sous pression réduite. Le résidu est repris par 30 ml d'un mélange dichlorométhane/méthanol 50/50. On élimine un insoluble par filtration sur célite. Le filtrat est évaporé sous pression réduite et le résidu est purifié par chromatographie sur gel de silice en éluant avec un mélange dichlorométhane/méthanol. Le solide obtenu est repris par l'éther diéthylique et recueilli par filtration, puis recristallisé dans 20 ml d'éthanol. On obtient 110 mg de composé.
PF = 222-223°C. RMN 1H (DMSO-d6, δ en ppm) : 2,75 (s, 3H) ; 4,6 (d, 2H) ; 5,25 (t, 1H) ; de 7,3 à 7,4 (m, 2H) ; de 7,45 à 7,55 (m, 3H) ; 7,6 (d, 1H) ; 7,7 (d, 2H) ; 7,75 (s, 1H) ; 7,85 (d, 2H) ; 8,5 (s, 1H). M+H = 315.

### Exemple 3 : Chlorhydrate (1:1) de {3-[2-(2,4-difluorophényl)-3-méthylimidazo[1,2-a]pyridin-6-yl]phényl}méthanol (composé 4 du tableau)

### 3.1 [3-(6-Aminopyridin-3-yl)phényl]méthanol

2,0 g de tétrakis(triphénylphosphine)palladium et 75 ml d'une solution 2M de carbonate de sodium sont ajoutés dans un ballon contenant une solution de 5,0 g de 5-bromopyridin-2-ylamine dans 140 ml de toluène, 5,7 g d'acide 3-(hydroxyméthyl)phénylboronique et 70 ml d'éthanol préalablement dégazée. On chauffe à 80°C pendant 16h. Après refroidissement, le mélange réactionnel est concentré sous pression réduite. Le résidu est repris entre l'eau et l'acétate d'éthyle. La phase organique est séparée, séchée et concentrée sous pression réduite. Le résidu est purifié par chromatographie sur gel de silice par un mélange dichlorométhane/méthanol. On obtient 4,99 g de composé.
RMN 1H (DMSO-d6, δ en ppm) : 4,55 (d, 2H) ; 5,2 (t, 1H) ; 6,05 (s, 2H) ; 6,55 (d, 1H) ; 7,2 (d, 1H) ; de 7,3 à 7,55 (m, 3H) ; 7,7 (d, 1H) ; 8,25 (s, 1H) ; M+H = 201.

### 3.2 5-[3-(tert-Butyldiméthylsilanyloxyméthyl)phényl]pyridin-2-ylamine

4,99 g de [3-(6-aminopyridin-3-yl)phényl]méthanol sont placés dans un ballon est dissous dans 240 ml de tétrahydrofurane. On y ajoute 2,2 g d'1*H*-imidazole puis 4,51 g de *tert*-butylchlorodiméthylsilane et on laisse agiter à température ambiante pendant 48 heures. Le mélange réactionnel est ensuite hydrolysé avec de l'eau et la phase organique, extraite à l'acétate d'éthyle, est séparée, séchée sur sulfate de magnésium et concentrée sous pression réduite. Le résidu obtenu est purifié par chromatographie sur gel de silice en éluant avec un mélange heptane/acétate d'éthyle. On obtient 7,0 g de composé.
RMN 1H (DMSO-d6, δ en ppm) : 0,09 (s, 6H) ; ; 0,91 (s, 9H) ; 4,75 (s, 2H) ; 6,02 (m, 2H) ; 6,52 (dd, 1H) ; 7,21 (d, 1H) ; 7,36 (t, 1H) ; 7,43 (d, 1H) ; 7,47 (s, 1H) ; 7,66 (dd, 1H) ; 8,21 (d, 1H). M+H = 315 ; PF = 82 - 84°C.

### 3.3 6-[3-(tert-Butyldiméthylsilanyloxyméthyl)phényl]-2-(2,4-difluorophényl)-3-méthylimidazo[1,2-a]pyridine

300 mg de 5-[3-(*tert*-butyldiméthylsilanyloxyméthyl)phényl]pyridin-2-ylamine dissous dans 5 ml d'éthanol sont placés dans un réacteur à vis. On y ajoute 200 mg d'hydrogénocarbonate de sodium et 470 mg de 2-bromo-1-(2,4-difluorophényl)propan-1-one. Le réacteur est fermé et chauffé à 80°C pendant 20 h. Après refroidissement, le élange réactionnel est concentré sous pression réduite. Le résidu obtenu est purifié par chromatographie sur gel de silice en éluant avec un mélange heptane/acétate d'éthyle. On obtient 220 mg de composé.
RMN 1H (CDCl₃, δ en ppm) : 0 (s, 6H) ; 0,85 (s, 9H) ; 2,4 (s, 3H) ; 4,7 (s, 2H) ; de 6,75 à 6,95 (m, 2H) ; de 7,2 à 7,35 (m, 4H) ; 7,45 (s, 1H) ; de 7,55 à 7,65 (m, 2H) ; 7.95 (s, 1H). M+H = 465.

### 3.4 {3-[2-(2,4-Difluorophényl)-3-méthylimidazo[1,2-a]pyridin-6-yl]phényl}méthanol

On place dans un ballon 210 mg de 6-[3-(*tert-*butyldiméthylsilanyloxyméthyl)phényl]-2-(2,4-difluorophényl)-3-méthylimidazo[1,2-*a*]pyridine dans 5 ml de tétrahydrofurane et on y ajoute 240 mg de fluorure de tétrabutylammonium. On agite à température ambiante pendant 48 h. Le mélange réactionnel est ensuite concentré sous pression réduite. Le résidu est purifié par chromatographie sur gel de silice en éluant avec un mélange dichlorométhane/méthanol. On obtient 130 mg de composé.
M+H = 351.

### 3.5 Chlorhydrate (1:1) de {3-[2-(2,4-difluorophényl)-3-méthylimidazo[1,2-a]pyridin-6-yl]phényl}méthanol

Une solution de 130 mg de {3-[2-(2,4-difluorophényl)-3-méthylimidazo[1,2-*a*]pyridin-6-yl]phényl}méthanol dans du dichloromé et du méthanol est passée sur firitté et on ajoute au filtrat 3,7 ml d'une solution 0,1N d'acide chlorhydrique dans l'isopropanol. Le mélange réactionnel est ensuite concentré sous pression réduite. Le résidu obtenu est trituré dans l'éther diéthylique et recueilli par filtration puis séché à l'étuve sous pression réduite. On obtient 128 mg de composé.
PF = 246 - 249°C. RMN 1H (DMSO-d6, δ en ppm) : 2,65 (s, 3H) ; 4,65 (s, 2H) ; de 7,35 à 7,65 (m, 4H) ; de 7,75 à 7,85 (m, 3H) ; 8,05 (d, 1H) ; 8,25 (d, 1H) ; 8,95 (s, 1H).M+H = 351.

### Exemple 4 : 2-{3-[2-(4-Chlorophényl)-3-méthylimidazo[1,2-a]pyridin-6-yl]phényl}propan-2-ol (composé 12 du tableau)

### 4.1 2-(3-bromophényl)propan-2-ol

Sous courant d'argon, 6,5 g de 3-bromoacétophénone sont placés dans un ballon et dissous dans 544 ml d'éther diéthylique et 272 ml de tétrahydrofurane. On refroidit à 0°C par un bain de glace et on y ajoute goutte à goutte 100 ml d'une solution 1M de bromure de méthylmagnésium dans l'éther dibutylique. On agite le mélange à 0°C pendant 1h et on ajoute 400 ml d'une solution aqueuse saturée de chlorure d'ammonium. La phase organique est séparée, séchée sur sulfate de magnésium et concentrée sous pression réduite. On obtient 10,0 g de composé.
RMN 1H (DMSO-d6, δ en ppm) : 1,45 (s, 6H) ; 5,15 (s, 1H) ; 7,25 (t, 1H) ; 7,4 (d, 1H) ; 7,45 (d, 1H) ; 7,65 (s, 1H).

### 4.2 2-[3-(6-Aminopyridin-3-yl)phényl]propan-2-ol

Sous courant d'argon, on place dans un ballon, 9,0 g de composé obtenu à l'étape 4.1, 11,05 g de 5-(4,4,5,5-tétraméthyl-1,3,2-dioxaborolan-2-yl)pyridin-2-ylamine, 83,7 ml d'une solution 2M de carbonate de sodium et 1,70 g de tétrakis(triphénylphosphine)palladium que l'on dissout dans 523 ml de N,N-diméthylformamide. Le mélange est chauffé 1h 30 à 80°C. Après refroidissement à température ambiante, 11 d'acétate d'éthyle est ajouté au milieu réactionnel que l'on filtre sur célite. La phase organique est ensuite séparée, lavée 3 fois par une solution de chlorure de sodium saturée, séchée sur sulfate de magnésium et concentrée sous pression réduite. Le résidu est purifié par chromatographie sur gel de silice en éluant par un mélange dichlorométhane/méthanol. Le solide obtenu est trituré dans l'éther diisopropylique, recueilli par filtration puis séché à l'étuve sous pression réduite. On obtient 2,35 g de composé.
RMN 1H (DMSO-d6, δ en ppm): 1,45 (s, 6H), 5,0 (s, 1H) ; 6,0 (s, 2H) ; 6,55 (d, 1H) ; de 7,3 à 7,4 (m, 3H) ; 7,65 (s, 1H) ; 7,7 (d, 1H) ; 8,25 (s, 1H).

### 4.3 2-{3-[2-(4-Chlorophényl)-3-méthylimidazo[1,2-a]pyridin-6-yl]phényl}propan-2-ol

58,8 mg (0,7 mmole) de bicarbonate de sodium sont pesés dans un tube à microondes. On y ajoute 57 mg (0,25 mmole) du composé obtenu en 4.2 en solution dans 2 ml de propan-1-ol, puis 92 mg (0,375 mmole) de 2-bromo-1-(4-chlorophényl)propan-1-one.en solution dans 1 ml de propan-1-ol Le tube est scellé puis irradié pendant 10 mn à 180°C. Le mélange réactionnel est refroidi à température ambiante, on y ajoute 200 mg de propanethiol sur silice (Biotage Si-Thiol) et le mélange est agité 6h à température ambiante, puis filtré. Le résidu est lavé par 2 fois 2 ml de propan-1-ol, puis le filtrat est évaporé et purifié par chromatographie. On obtient 26,4 mg de composé.
RMN 1H (DMSO-d6, δ en ppm) : 1,5 (s, 6H) ; 2,75 (s, 3H) ; 5,1 (s, 1 H) ; 7,45 (t, 1H) ; 7,5 (d, 1H) ; 7,55 (d, 2H) ; de 7,6 à 7,7 (m, 3H) ; de 7,8 à 7,9 (m, 3H) ; 8,6 (s, 1H). M+H =377.

### Exemple 5 : [2,6-Difluoro-3-(3-méthyl-2-phénylimidazo[1,2-a]pyridin-6-yl)phényl]méthanol (composé 17 du tableau)

### 5.1 (3-bromo-2,6-difluorophényl)méthanol

20 g de 3-bromo-2,6-difluorobenzaldéhyde sont mis en solution dans 450 ml de méthanol et refroidis au bain de glace; puis on y ajoute par portions 3,42 g de borohydrure de sodium. Le mélange est agite à température ambiante pendant 1 heure puis le solvant est évaporé sous pression réduite. Le résidu est repris entre l'eau et le dichlorométhane, la phase organique est séparée, séchée et concentrée sous pression réduite. Le résidu est cristallisé dans le n-pentane. On obtient 14,6 g de composé utilisé tel quel dans l'étape suivante.
Spectre RMN 1H (CDCl3, δ en ppm) :.2,0 (s, 1H) ; 4,9 (s, 2H) ; de 6,85 à 7,0 (m, 1H) ; de 7,5 à 7,65 (m, 1H).

### 5.2 (3-Bromo-2,6-difluorobenzyloxy)tert-butyldiméthylsilane

11, 15 g du composé obtenu en 5.1 sont dissous dans 150 ml de THF, on ajoute 5,1 g d'imidazole puis 9,04 g de chloro-*tert*-butyldiméthylsilane et agite le mélange à température ambiante pendant 24 heures. Puis on évapore le solvant, reprend le résidu et l'eau et l'éther diéthylique, décante, lave la phase organique à l'eau et la sèche sur sulfate de sodium. On évapore le solvant sous pression réduite. On obtient 17,5 g d'huile.
Spectre RMN 1H (CDCl3, δ en ppm) : 0,0 (s, 6H) ; 0,8 (s, 9H) ; 4,65 (s, 2H) ; de 6,65 à 6,7 (m, 1H) ; de 7,3 à 7,4 (m, 1H).

### 5.3 5-[3-(tert-Butyldiméthylsilanyloxyméthyl)-2,4-difluorophényl]pyridin-2ylamine

6,7 g du composé obtenu en 5.2, 4,40 g de 5-(4,4,5,5-tétraméthyl-1,3,2-dioxaborolan-2-yl)pyridin-2-ylamine, 30 ml d'une solution 2M de carbonate de sodium et 10,816 mg de [1,1'-bis(diphenylphosphino)ferrocène]dichloropalladium sont dissous dans 80 ml de *N*,*N*-diméthylformamide et placés dans un ballon sous courant d'argon. Le mélange est chauffé 2h à 80°C. Après refroidissement à température ambiante, on évapore les solvants sous pression réduite et reprend le résidu entre l'eau et l'acétate d'éthyle et élimine un insoluble par filtration sur célite. La phase organique est décantée, lavée avec une solution aqueuse saturée en chlorure de sodium et séchée sur sulfate de sodium. Le composé est purifié par chromatographie en éluant avec un mélange de dichlorométhane et de méthanol. On obtient 4,25 g de solide blanc.

Spectre RMN 1H (DMSO-d6, δ en ppm) : 0 (s, 6H) ; 0,8 (s, 9H), 4,4 (s, 2H); 6,05 (s, 2H) ; 6,45 (d, 1) ; 7,05 (t, 1) ; de 7,35 à 7,45 (m, 2H) ; 8,0 (s, 1H). M+H = 351

### 5.4 6-[3-(tert-Butyldiméthylsilanyloxyméthyl)-2,4-difluorophényl]-3-methyl-2-phénylimidazo[1,2-a]pyridine

En procédant comme dans l'exemple 4.3 en partant de 0,25 mmole du composé obtenu en 5.3 et de 0,375 mmole de 2-bromo-1-phénylpropan-1-one, on obtient le composé attendu, utilisé tel quel pour l'étape suivante.

### 5.5 [2,6-Difluoro-3-(3-méthyl-2-phénylimidazo[1,2-a]pyridin-6-yl)phényl] méthanol

Le composé brut obtenu en 5.4 est dissous dans 5 ml de THF contenant 0,5 mmole de fluorure de tetrabutylammonium hydraté. Le mélange est agité 16h à température ambiante, puis le solvant est évaporé sous pression réduite. Le composé est purifié par chromatographie. On obtient 22 mg de composé.
RMN 1H (DMSO-d6, δ en ppm) : 2,7 (s, 3H) ; 4,6 (s, 2H) ; 5,35 (s, 1H) ; 7,25 (t, 1H) ; de 7,35 à 7,45 (m, 2H) ; 7,5 (t, 2H) ; de 7,65 à 7,7 (m, 2H) ; 7,85 (d, 2H) ; 8,45 (s, 1H). M+H = 351.

### Exemple 6: 2-{3-[3-Chloro-2-(4-chlorophényl)imidazo[1,2-a]pyridin-6-yl]phényl}propan-2-ol (composé 23 du tableau)

### 6.1 6-Bromo-2-(4-chlorophényl)imidazo[1,2-a]pyridine

Dans un tricol de 250 mL, muni d'un agitateur magnétique et maintenu sous atmosphère d'azote sont introduits 2,5 g (14,45 mmoles) de 2-amino-5-bromopyridine, 3,37 g (14,45 mmoles) de 2-bromo-4'-chloroacetophenone et 1,82 g (21,67 mmoles) d'hydrogénocarbonate de sodium et 110 de 1-propanol. Après 18 heures d'agitation à 80°C, le mélange réactionnel ramené à température ambiante est dilué avec 400 mL d'eau. Le précipité formé est recueilli par filtration, lavé à l'eau puis séché à 80°C sous pression réduite. On isole ainsi 3,96 g de produit attendu sous la forme d'une poudre beige.
PF=210-211°C
R.M.N. ¹H (DMSO D₆), δ (ppm) : 7,4 (d, 1H) ; 7,5 (d, 2H) ; 7,6 (d, 1H) ; 8,0 (d, 2H) ; 8,4 (s, 1H) ; 8,95 (s, 1H).

### 6.2 6-Bromo-3-chloro-2-(4-chlorophényl)imidazo[1,2-a]pyridine

A une suspension de 1,0 g (3,25 mmoles) de composé obtenu à l'étape 6.1 dans 100 mL de méthanol, maintenue sous atmosphère inerte, est ajouté 0,52 g (3,90 mmoles) de *N-*chlorosuccinimide. Le mélange réactionnel est agité à température ambiante 2 h. Après ce temps, le précipité formé est recueilli par filtration, lavé avec du méthanol puis séché à 80°C sous pression réduite. On isole 0,94 g du produit attendu sous la forme d'une poudre beige.
PF=183-185°C
R.M.N. ¹H (DMSO D₆), δ (ppm) 7,55 (d, 1H) ; 7,6 (d, 2H) ; 7,7 (d, 1H) ; 8,15 (d, 2H) 8,7 (s, 1H).

### 6.3 1-{3-[3-Chloro-2-(4-chlorophényl)imidazo[1,2-a]pyridin-6-yl]phényl}éthanone

Dans un tricol de 100 mL, muni d'un agitateur magnétique et maintenue sous atmosphère inerte sont introduits 15 mL d'acétonitrile, 15 mL de toluène et 15 mL d'une solution aqueuse 2M de carbonate de sodium. A cette solution sont ajoutés 0,8 g (2,34 mmoles) de composé obtenu selon le protocole décrit à l'étape 6.2, 0,49 g (3,04 mmoles) d'acide 3-acétylphénylboronique et 130 mg (0,12 mmole) de tétrakis(triphénylphosphine)palladium. Après 4 heures d'agitation à 80°C, le mélange réactionnel ramené à température ambiante est concentré au tiers sous pression réduite, dilué avec 100 mL d'eau puis extrait par trois fois 40 mL d'acétate d'éthyle. Les phases organiques rassemblées sont lavées avec deux fois 30 mL d'eau, séchées sur du sulfate de sodium, filtrées et concentrées sous pression réduite. On purifie le solide résultant par chromatographie sur colonne de gel de silice en éluant avec un mélange d'heptane et d'acétate d'éthyle. On isole 0,69 g du produit attendu.
PF = 186-188°C
R.M.N. ¹H (DMSO D₆), δ (ppm) : 2,7 (s, 3H) ; 7,6 (d, 2H) ; 7,7 (m, 1H) ; 7,85 (s, 2H) ; 8,05 (d, 1H) ; 8,15 (d, 1H) ; 8,2 (d, 2H) ; 8,35 (s, 1H) ; 8,7 (s, 1H).

### 6.4 2-{3-[3-Chloro-2-(4-chlorophényl)imidazo[1,2-a]pyridin-6-yl]phényl}propan-2-ol

Dans un tricol de 250 mL, préalablement séché à l'étuve, muni d'un agitateur magnétique et maintenue sous atmosphère inerte est ajouté 0,4 g (1,05 mmole) de composé obtenu à l'étape 6.3, dissous dans un mélange de 100 mL de diéthyléther anhydre et de 75 mL de THF anhydre. A cette solution refroidie à 0°C est ajoutée goutte à goutte 1,05 mL (2,10 mmoles) d'une solution 2M de bromure de méthylmagnésium dans l'éther diéthylique. Le mélange réactionnel est agité à 0°C pendant quatre heures puis hydrolysé par ajout sous agitation vigoureuse de 100 mL d'une solution de chlorure d'ammonium (30% aqueuse). On extrait le produit avec trois fois 50 mL d'acétate d'éthyle. On lave les phases organiques rassemblées avec deux fois 20 mL d'eau, on sèche sur sulfate de sodium et on concentre sous pression réduite. On purifie le produit résultant par chromatographie sur colonne de gel de silice en éluant avec un mélange d'heptane et de dichlorométhane. On isole ainsi 148 mg du produit attendu.
PF = 172-173°C
R.M.N. ¹H (DMSO D₆), δ (ppm): 1,45 (s, 6H) ; 5,05 (s, 1H) ; de 7,3 à 7,6 (m, 7H) ; 7,7 (m, 2H) ; 7,8 (s, 1H) ; 8,1 (d, 2H) ; 8,45 (s, 1H).

### Exemple 7 : 2-{3-[2-(4-Chlorophényl)-3-fluoroimidazo[1,2-a]-pyridin-6-yl]phényl}propan-2-ol (composé 22 du tableau)

### 7.1 1-{3-[2-(4-Chlorophényl)imidazol[1,2-a]pyridin-6-yl]phényl}éthanone

Ce composé a été préparé selon un procédé similaire à celui décrit à l'étape 6.3 en faisant réagir 5 g (16,26 mmoles) de 6-bromo-2-(4-chlorophényl)imidazo[1,2-a]pyridine préparé selon le protocole décrit à l'étape 6.1 avec 3,46 g (21,13 mmoles) d'acide 3-acétylphénylboronique, en présence d'une solution aqueuse 2M de carbonate de sodium (90 mL) et de 0,94 g (0,81 mmole) de tétrakis(triphénylphosphine)palladium dans 180 mL d'un mélange d'acétonitrile et de toluène (v/v). On obtient 5,37 g du produit attendu.

PF = 173 - 175°C.

### 7.2 2-{3-[2-(4-Chlorophényl)imidazo[1,2-a]pyridin-6-yl]phényl}propan-2-ol

Ce composé a été préparé selon un procédé similaire à celui décrit à l'étape 6.4 en faisant réagir 2 g (5,77 mmoles) de composé préparé selon le protocole décrit l'étape 7.1 avec 2,06 g (17,30 mmoles) d'une solution de bromure de méthylmagnésium à 0°C dans 250 mL d'un mélange d'éther éthylique et de tétrahydrofuran (v/v). On obtient 1,45 g du produit attendu. R.M.N. ¹H (DMSO D₆), δ (ppm) : 1,45 (s, 6H) ; 5,1 (s, 1H) ;. 7,35-7,7 (m, 7H) ; 7,8 (s, 1H) ; 8,0 (d, 2H) ; 8,45 (s, 1H), 8,85 (s, 1H)

### 7.3 2-{3-[2-(4-Chlorophényl)-3-fluoroimidazo[1,2-a]pyridin-6-yl]phényl}propan-2-ol

A une suspension, agitée à 0°C sous atmosphère inerte, de 0,4 g (1, 10 mmole) de composé préparé à l'étape 7.2, dans 40 mL d'acétonitrile, est ajoutée goutte à goutte (15 minutes), 0,195 g (1,10 mmole) de 1-chlorométhyl-4-fluoro-1,4-diazoniabicyclo[2.2.2]octane bis-tétrafluoroborate ("selectfluor") en solution dans 10 mL d'un mélange de THF/eau (v/v). Après 18 heures d'agitation à température ambiante le mélange réactionnel est concentré sous pression réduite. Le produit obtenu est purifié par chromatographie sur colonne de silice en éluant avec un mélange d'heptane et d'acétate d'éthyle. Le solide obtenu après évaporation du solvant sous pression réduite est séché à l'étuve à 80°C pour donner 82 mg du produit attendu.
PF=174-175°C
R.M.N. ¹H (DMSO D₆), δ (ppm) : 1,45 (s, 6H) ; 5,1 (s, 1H) ; 7,4-7,7 (m, 7H) ; 7,9 (s, 1H) 8,0 (d, 2H) ; 8,6 (s, 1H).

### Exemple 8 : {3-[3-Chloro-2-(4-chlorophényl)imidazo[1,2-a]pyridin-6-yl]phényl}méthanol (composé 21 du tableau)

### 8.1 {3-[2-(4-Chlorophényl)imidazo[1,2-a] pyridin-6-yl] phényl} méthanol

Sous un courant d'azote, 210 mg de composé obtenu en 6.1, 155 mg d'acide 3-(hydroxyméthyl)phénylboronique et 24 mg de tétrakis(triphénylphosphine)palladium sont placés dans un tube à micro-ondes contenant 3 ml de toluène préalablement dégazé sous courant d'azote, 3 ml d'acétonitrile et 3 ml d'une solution 2M de carbonate de sodium. Le tube est placé dans un appareil à micro-ondes et irradié à 150°C pendant 15 mn. La phase organique est séparée, séchée et le filtrat est concentré sous pression réduite. Le résidu obtenu est repris par du dichlorométhane. On recueille le précipité par filtration, le lave au dichlorométhane et le sèche au dessicateur sous pression réduite. On obtient 175 mg de composé.
PF = 181 - 182°C. RMN 1H (DMSO-d6, δ en ppm) : 4,57 (d, J = 5,5Hz, 2H) ; 5,23 (t, J = 5,6Hz, 1H) ; de 7,28 à 7,71 (m, 8H) ; 7,97 (m, J = 8,5Hz, 2H) ; 8,41 (s, 1H) ; 8,84 (m, 1H). M+H = 335.

### 8.2 {3-[3-Chloro-2-(4-chlorophényl)imidazo[1,2-a]pyridin-6-yl]phényl}méthanol

Le composé N° 21 a été préparé selon un procédé similaire à celui décrit à l'étape 6.2 en faisant réagir 100 mg (0,3 mmole) de composé préparé selon le protocole décrit à l'étape 8.1 avec 52 mg (0,39 mmole) de N-chlorosuccinimide dans 5 mL de méthanol. On obtient 75 mg du produit attendu.
PF: 195-197°C
R.M.N. ¹H (DMSO D₆), δ (ppm): 4,6 (d, 2H) ; 5,25 (t, 1H) ; 7,4 (d, 1H) ; 7,5 (t, 1H) ; de 7,65 à 7,8 (m, 6H) ; 8,15 (m, 2H) ; 8,55 (s, 1H).

### Exemple 9 : 3-Chloro-6-(3-méthoxyméthylphényl)-2-(4-chlorophenyl)imidazo[1,2-a]pyridine (composé 28 du tableau)

Ce composé a été préparé selon un procédé similaire à celui décrit à l'étape 6.3 en faisant réagir 0.4 g (1,17 mmole) de composé préparé selon le protocole décrit à l'é pe 6.2 avec 0,25 g (1,52 mmole) d'acide 3-méthoxyméthylphénylboronique, en présence d'une solution aqueuse 2M de carbonate de sodium (10 mL) et de 67 mg (0,06 mmole) de tétrakis(triphénylphosphine)palladium dans 20 mL d'un mélange d'acétonitrile et de toluène (v/v). On obtient 0,31 g du produit attendu après purification.
PF : 120 - 122°C
R.M.N. ¹H (DMSO D₆), δ (ppm) : 3,4 (s, 3H) ; 4,5 (s, 2H) ; 7,4 (m, 1H) ; 7,55 (m, 1H) ; ; 7,65 (d, 2H) ; de 7,75 à 7,85 (m, 4H) ; 8,15 (d, 2H) ; 8,6 (s, 1H),

Les tableaux qui suivent illustrent les structures chimiques de formule générale (I) (tableau 2) et les caractéristiques physicochimiques (tableau 3) de quelques exemples de composés selon l'invention. Dans ces tableaux :
- la colonne « position » renseigne sur la position « , 2, 3, ou 4» de substitutiondu groupe 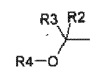 sur le noyau phényle;
- « Ph » signifie phényle
- « Cl » signifie chlore ;
- « F » signifie fluor ;
- « Me » signifie méthyle ;
- « MeO » signifie méthoxy ;
- « (F₂CH)O » signifie difluorométhoxy ;
- dans la colonne R, le chiffre devant le substituant renseigne la position de sustitution du groupe R sur le noyau phényle;
- dans la colonne X, le chiffre devant le substituant renseigne la position de sustitution du groupe X sur le noyau imidazo[1,2-*a*]pyridine ;
- dans la colonne « Sel/base », « - » représente un composé sous forme de base libre, alors que « HCl » représente un composé sous forme de chlorhydrate et le rapport entre parenthèses est le rapport (acide :base) ;
- la colonne « PF » renseigne les points de fusion des produits en degrés Celsius (°C) ou, lorsque les produits ont été isolés sous la forme de solide amorphe ou d'huile, ils sont caractérisés par leur masse [M+H].

**Tableau 2**

| | | | | | | | | |
|---|---|---|---|---|---|---|---|---|
| 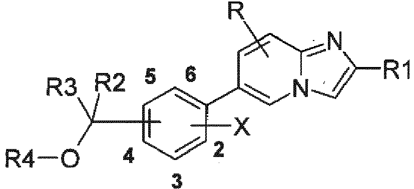 | | | | | | | | |

| **Ex** | **R** | **R₁** | **position** | **X** | **R₂** | **R₃** | **R₄** | **Sel/base** |
|---|---|---|---|---|---|---|---|---|
| **1** | 8-Me | 4-Cl-Ph | 3 | H | H | H | H | - |
| **2** | 7-Me | 4-Cl-Ph | 3 | H | H | H | H | - |
| **3** | 3-Me | Ph | 3 | H | H | H | H | - |
| **4** | 3-Me | 2,4-(F)₂-Ph | 3 | H | H | H | H | HCl (1:1) |
| **5** | 3-Me | 3-Me-4-Cl-Ph | 3 | H | H | H | H | HCl (1 : 1) |
| **6** | 3-Me | 4-Cl-Ph | 3 | H | H | H | H | HCl (1 :1) |
| **7** | 3-Me | 4-MeO-Ph | 3 | H | H | H | H | HCl (1 :1) |
| **8** | 3-Me | 4-(F₂CH)O-Ph | 3 | H | H | H | H | HCl(1:1) |
| **9** | 8-Me | 2-Naphthyle | 3 | H | H | H | H | - |
| **10** | 8-Me | 2-Naphthyle | 4 | H | H | H | H | - |
| **11** | 3-Me | Ph | 3 | H | Me | Me | H | - |
| **12** | 3-Me | 4-Cl-Ph | 3 | H | Me | Me | H | - |
| **13** | 3-Me | 3-Cl-Ph | 3 | H | Me | Me | H | - |
| **14** | 3-Me | 2,4-diF-Ph | 3 | H | Me | Me | H | - |
| **15** | 3-Me | 4-MeO-Ph | 3 | H | Me | Me | H | - |
| **16** | 3-Me | 4-MeO-Ph | 2 | 3-F | H | H | H | - |
| **17** | 3-Me | Ph | 3 | 2,4-diF | H | H | H | - |
| **18** | 3-Me | 4-Cl-Ph | 3 | 2,4-diF | H | H | H | - |
| **19** | 3-Me | 3-Cl-Ph | 3 | 2,4-diF | H | H | H | - |
| **20** | 3-Me | 4-MeO-Ph | 3 | 2,4-diF | H | H | H | - |
| **21** | 3-Cl | 4-Cl-Ph | 3 | H | H | H | H | - |
| **22** | 3-F | 4-Cl-Ph | 3 | H | Me | Me | H | - |
| **23** | 3-Cl | 4-Cl-Ph | 3 | H | Me | Me | H | - |
| **24** | 3-Me | Ph | 2 | 3-F | H | H | H | - |
| **25** | 3-Me | 4-Cl-Ph | 2 | 3-F | H | H | H | - |
| **26** | 3-Me | 3-Cl-Ph | 2 | 3-F | H | H | H | - |
| **27** | 3-Me | 2,4-diF | 2 | 3-F | H | H | H | - |
| **28** | 3-Cl | 4-Cl-Ph | 3 | H | H | H | Me | - |
| **29** | 3-Cl | 4-Cl-Ph | 3 | 2,4-diF | H | H | H | - |

**Tableau 3**

| **Ex** | **RMN** Spectres RMN ¹H (DMSO-d6, *δ* en ppm) | **PF/[M+H]** |
|---|---|---|
| **1** | 2,6 (s, 3H) ; 4,6 (d, 2H) ; 5,3 (t, 1H) ; 7,3 (d, 1H) ; de 7,4 à 7,5 (m, 4H) ; 7,6 (d, 1H) ; 7,7 (s, 1H) ; 8,0 (d, 2H) ; 8,4 (s, 1H) ; 8, 7 (s, 1H). | 174-177 |
| **2** | 2,25 (s, 3H) ; 4,55 (d, 2H) ; 5,25 (t, 1H) ; 7,3 (d, 1H) ; de 7,35 à 7,55 (m, 6H) ; 8,0 (d, 2H) ; 8,35 (s, 1H) ; 8,4 (s, 1H). | 183-184 |
| **3** | 2,75 (s, 3H) ; 4,6 (d, 2H) ; 5,25 (t, 1H) ; de 7,3 à 7,4 (m, 2H) ; de 7,45 à 7,55 (m, 3H) ; 7,6 (d, 1H) ; 7,7 (d, 2H) ; 7,75 (s, 1H) ; 7,85 (d, 2H) ; 8,5 (s, 1H). | 222-223 |
| **4** | 2,65 (s, 3H) ; 4,65 (s, 2H) ; de 7,35 à 7,65 (m, 4H) de 7,75 à 7,85 (m, 3H) ; 8,05 (d, 1H) ; 8,25 (d, 1H) ; 8,95 (s, 1H). | 246-249 |
| **5** | 2,5 (s, 3H) ; 2,8 (s, 3H) ; 4,65 (s, 2H) ; 7,45 (d, 1H) ; 7,55 (t, 1H) ; de 7,65 à 7,75 (m, 2H) ; 7,8 (d, 1H) ; 7,85 (m, 2H) ; 8,05 (d, 1H) ; 8,25 (d, 1H) ; 9,0 (s, 1H) ; isomère majoritaire à 80 % ( l'isomère minoritaire à 20% étant le dérivé, 3-Cl-4- Me) | 272-275 |
| **6** | 2,8 (s, 3H) ; 4,65 (s, 2H) ; 7,45 (d, 1H) ; 7,55 (t, 1H) ; de 7,7 à 7,8 (m, 3H) ; de 7,82 à 7,9 (m, 3H) ; 8,05 (d, 1H) ; 8,25 (d, 1H) ; 9,0 (s, 1H). | 263-267 |
| **7** | 2,8 (s, 3H) ; 3,9 (s, 3H) 4,65 (s, 2H) ; 7,2 (m, 2H) ; 7,45 (d, 1H) ; 7,55 (t, 1H) ; 7,8 (m, 3H) ; 7,85 (s, 1H) ; 8,05 (d, 1H) ; 8,25 (d, 1H) ; 9,0 (s, 1H), | 260-263 |
| **8** | 2,9 (s, 3H) ; 4,7 (s, 2H) ; 7,3 (s, 1H) ; de 7,5 à 7,6 (m, 3H) ; 7,65 (m, 1H) ; 7,85 (d, 1H) ; 7,95 (s, 1H) ; 8,0 (d, 2H) ; 8,15 (d, 1H) ; 8,35 (d, 1H) ; 9,05 (s, 1H). | 259-261 |
| **9** | 2,65 (s, 3H) ; 4,6 (d, 2H) ; 5,2 (t, 1H) ; de 7,25 à 7,7 (m, 7H) ; de 7,85 à 8,15 (m, 4H) ; 8,5 (m, 2H) ; 8,75 (s, 1H). | 94-96 |
| **10** | 2,6 (s, 3H) ; 4,55 (d, 2H) ; 5,2 (t, 1H) ; de 7,4 à 7,6 (m, 5H) ; 7,65 ( d, 2H) ; de 7,85 à 8,05 (m, 3H) ; 8,1 (d, 1H) ; 8,5 (s, 1H) ; 8,55 (s, 1H); 8,7 (s, 1H). | 198-200 |
| **11** | 1,5 (s, 6H) ; 2,75 (s, 3H) ; 5,1 (s, 1H) ; de 7,4 à 7,55 (m, 5H) ; 7,65 (d, 1H) ; 7,75 (s, 2H) ; de 7,8 à 7,9 (m, 3H) ; 8,6 (s, 1H). | [343] |
| **12** | 1,5 (s, 6H) ; 2,75 (s, 3H) ; 5,1 (s, 1H) ; 7,45 (t, 1H) ; 7,5 (d, 1H) ; 7,55 (d, 2H) ; de 7,6 à 7,7 (m, 3H) ; de 7,8 à 7,9 (m, 3H) ; 8,6 (s, 1H). | [377] |
| **13** | 1,5 (s, 6H) ; 2,75 (s, 3H) ; 5,1 (s, 1H) ; de 7,4 à 7,55 (m, 4H) ; de 7,6 à 7,65 (m, 3H) ; 7,8 (d, 1H) ; 7,9 (d, 2H) ; 8,55 (s, 1H). | [377] |
| **14** | 1,5 (s, 6H) ; 2,55 (s, 3H) ; 5,1 (s, 1H) ; de 7,2 à 7,25 (m, 1H) ; de 7,35 à 7,55 (m, 3H) ; de 7,6 à 7,75 (m, 4H) ; 7,85 (t, 1H) ; 8,5 (s, 1H). | [379] |
| **15** | 1,5 (s, 6H) ; 2,75 (s, 3H) ; 3,85 (s, 3H) ; 5,1 (s, 1H) ; 7,15 (m, 2H) 7,45 (t, 1H) ; 7,55 (d, 1H) ; 7,65 (d, 1H) ; de 7,75 à 7,9 (m, 5H) ; 8,65 (s, 1H). | [373] |
| **16** | 2,65 (s, 3H) ; 3,8 (s, 3H) ; 4,45 (s, 2H) ;5,35 (s, 1H) ; 7,05 (d, 2H) ; de 7,25 à 7,35 (m, 2H) ; 7,4 (d, 1H) ; de 7,45 à 7,5 (m, 1H) ; 7,55 (d, 1H) ; 7,75 (d, 2H) ;8,45 (s, 1H). | [363] |
| **17** | 2,7 (s, 3H) ; 4,6 (s, 2H) ; 5,35 (s, 1H) ; 7,25 (t, 1H) ; de 7,35 à 7,45 (m, 2H) ; 7,5 (t, 2H) ; de 7,65 à 7,7 (m, 2H) ; 7,85 (d, 2H) ; 8,45 (s, 1H). | [351] |
| **18** | 2,7 (s, 3H) ; 4,6 (s, 2H) ; 5,35 (s, 1H) ; 7,25 (t, 1H) ; 7,4 (d, 1H) ; 7,55 (d, 2H) ; de 7,65 à 7,7 (m, 2H) ; 7,85 (d, 2H) ; 8,5 (s, 1H). | [385] |
| **19** | 2,7 (s, 3H) ; 4,6 (s, 2H) ; 5,35 (s, 1H) ; 7,25 (t, 1H) ; 7,4 (d, 2H) ; 7,5 (t, 1H) ; de 7,65 à 7,7 (m, 2H) ; 7,8 (d, 1H) ; 7,85 (s, 1H) ; 8,5 (s, 1H), | [385] |
| **20** | 2,65 (s, 3H) ; 3,8 (s, 3H) ; 4,6 (s, 2H) ; 5,35 (s, 1H) ; 7,05 (d, 2H) ; 7,25 (t, 1H) ; 7,4 (d, 1H) ; de 7,6 à 7,7 (m, 2H) ; 7,75 (d, 2H) ; 8,45 (s, 1H). | [381] |
| **21** | 4,6 (d, 2H) ; 5,25 (t, 1H) ; 7,4 (d, 1H) ; 7,5 (t, 1H) ; de 7,65 à 7,8 (m, 6H) ; 8,15 (m, 2H) ; 8,55 (s, 1H). | 195-197 |
| **22** | 1,45 (s, 6H) ; 5,1 (s, 1H) ;. de 7,4 à 7,7 (m, 7H) ; 7,9 (s, 1H) ; 8,0 (d, 2H) ; 8,6 (s, 1H). | 174-175 |
| **23** | 1,45 (s, 6H) ; 5,05 (s, 1H) ; de 7,3 à 7,6 (m, 7H) ; 7,7 (m, 2H) ; 7,8 (s, 1H) ; 8,1 (d, 2H) ; 8,45 (s, 1H). | 172-173 |
| **24** | 2,7 (s, 3H) ; 4,45 (d, 2H) ; 5,35 (t, 1H) ; de 7,25 à 7,55 (m, 7H) ; 7,65 (d, 1H) ; 7,85 (d, 2H) ; 8,5 (s, 1H). | [333] |
| **25** | 2,65 (s, 3H) ; 4,45 (d, 2H) ; 5,35 (t, 1H) ; de 7,25 à 7,35 (m, 2H) ; de 7,45 à 7,6 (m, 4H) ; 7,65 (d, 1H) ; 7,85 (d, 2H) ; 8,5 (s, 1H). | [367] |
| **26** | 2,7 (s, 3H) ; 4,45 (d, 2H) ; 5,35 (t, 1H) ; de 7,25 à 7,35 (m, 2H) ; de 7,4 à 7,55 (m, 4H) ; 7,7 (d, 1H) ; 7,8 (d, 1H) ; 7,85 (s, 1H) ; 8,5 (s, 1H). | [367] |
| **27** | 2,55 (s, 3H) ; 4,45 (d, 2H) ; 5,35 (t, 1H) ; de 7,2 à 7,55 (m, 6H) ; de 7,65 à 7,8 (m, 2H) ; 8,5 (s, 1H). | [369] |
| **28** | 3,4 (s, 3H) ; 4,5 (s, 2H) ; 7,4 (m, 1H) ; 7,55 (m, 1H) ; 7,65 (d, 2H) ; de 7,75 à 7,85 (m, 4H) ; 8,15 (d, 2H) ; 8,6 (s, 1H). | 120-122 |
| **29** | 4,1(d, 2H) ; 5,35 (t, 1H) ; 7,25 (m, 1H) ; 7,55 (m, 3H) ; 7,7 (m, 1H) ; 7,85 (d, 1H) ; 8,15 (d, 2H) ; 8,5 (s, 1H). | 201-203 |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 10 µM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 1, 3, 10, 15 et 19 ont montré une EC₅₀ de respectivement 5 ; 0,5 ; 68 ; 161 et 1,7 nM. Il apparaît donc que les composés selon l'invention ont un effet modulateur de NOT.

Les composés selon l'invention peuvent donc être utilisés our la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie, les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcereuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 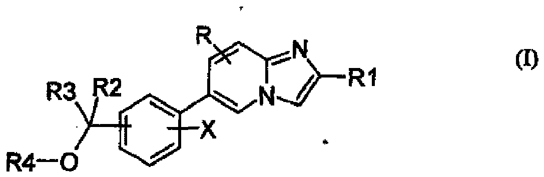 dans laquelle ;
R₁ représente un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, (C₁-C₁₀)alcoxy, halo(C₁-C₁₀)alcoxy,
X représente de 1 à 2 substituants identiques ou différents l'un de l'autre choisis parmi hydrogène, halogène;
R représente en position 3, 7 ou 8 de l'imidazo[1,2-*a*]pyridine de 1 à 2 substituants identiques ou différents l'un de l'autre choisis parmi un halogène, (C₁-C₁₀)alkyle;
R₂ et R₃ représentent, indépendamment l'un de l'autre,
un atome d'hydrogène,
un groupe (C₁-C₁₀)alkyle;
R₄ représente :
un atome d'hydrogène,
un groupe (C₁-C₁₀)alkyle, à l'état de base ou de sel d'addition à un acide.

2. Composés de formule (I) selon la revendication 1, **caractérisés en ce que** :
Le groupe 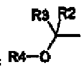 est en position 2, 3 ou 4 sur le noyau phényle qui le porte.

3. Composés de formule (I) selon la revendication 1 ou 2, **caractérisés en ce que**
R₁ représente un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₁₀)alkyle, (C₁-C₁₀)alcoxy, halo(C₁-C₁₀)alcoxy;
X représente un atome d'hydrogène,
R est en position 3, 7 ou 8 de l'imidazo[1,2-*a*]pyridine et représente un (C₁-C₁₀)alkyle ;
R₂ et R₃ représentant, indépendamment l'un de l'autre, un atome d'hydrogène ;
R₄ représente un atome d'hydrogène,
à l'état de base ou de sel d'addition à un acide.

4. Composés
{3-[2-(4-Chlorophényl)-8-méthylmido[1,2-*a*]pyridin-6-yl]phényl}methanol ;
{3-[2-(4-Chlorophényl)-7-méthylimidazo[1,2-*a*]pyridin-6-yl]phényl}méthanol ;
[3-((3-Méthyl-2-phénylimidazo[1,2-a]pyridin-6-yl) phényl]méthanol ;
{3-[2-(2,4-diflurophényl)-3-méthylimidazo[1,2-*a*]pyridin-6-yl]phényl}méthanol et son chlorhydrate ;
{3-[2-(4-chloro-3-méthylphényl)-3-méthylimidazo[1,2-*a*]pyridin-6-yl]phényl}méthanol et son chlorhydrate ;
{3-[2-(4-chlorophényl)-3-méthylimidazo[1,2-*a*]pyridin-6-yl]phényl}méthanal et son chlorhydrate ;
{3-[2-(4-méthoxyphényl)-3-méthylimidazo[1,2-*a*]pyridin-6-yl]phényl}méthanol et son chlorhydrate ;
{3-[2-[4-(difluorométhyloxy)phényl]-3-méthylimidazo[1,2-*a*]pyridin-6-yl]phényl}méthanol et son chlorhydrate ;
[3-((8-Méthyl-2-naphthyl-2-ylimidazo[1,2-*a*]pyridin-6-yl)phényl]méthanal ;
[4-((8-Méthyl-2-naphthyl-2-ylimidazo[1,2-*a*]pyridin-6-yl)phenyl]méthanol ;
2-[3-(3-Méthyl-2-phénylimidazo[1,2-*a*]pyridin-6-yl)phényl]proppan-2-ol ;
2-{3-[2-(4-Chlorophényl)-3-méthylimidazo[1,2-*a*]pyridin-6-yl]phényl}propan-2-ol;
2-{3-[2-(3-Chlorophényl)-3-méthylimidazo[1,2-*a*]pyridin-6-yl]phényl}propan-2-ol;
2-{3-[2-(2,4-Diflurorophényl)-3-méthylimidazo[1,2-*a*]pyridin-6-yl]phényl}propan-2-ol ;
2-{3-[2-(4-Méthoxyphényl)-3-méthylimidazo[1,2-*a*]pyridin-6-yl]phényl}propan-2-ol;
{2-Fluoro-6-[2-(4-méthoxyphényl)-3-méthylimidazo[1,2-*a*]pyridin[1,2-a]pyridin-6-yl]phényl}méthanol;
[2,6-Difluoro-3-(3-méthyl-2-phénylimidazo[1,2-*a*]pyridin-6-yl)phényl]méthanol;
{3-[2-(4-Chlorophényl)-3-méthylimidazo[1,2-*a*]pyridin-6-yl]-2,6-difluorophényl}méthanol;
{3-[2-(3-Chlorophényl)-3-méthylimidazo[1,2-*a*]pyridin-6-yl]-2,6-difluorophényl}méthanol ;
{2,6-Difluoro-3-[2-(4-méthoxyphényl)-3-méthylimidazo[1,2-*a*]pyridin-6-yl]-phényl}méthanol ;
{3-[3-Chloro-2-(4-chlorophényl)imidazo[1,2-*a*]pyridin-6-yl]phényl}méthanol ;
2-{3-[2-(4-Chlorophényl)-3-fluoroimidazo[1,2-*a*]pyridin-6-yl]phényl}propan-2-ol ;
2-{3-[3-Chloro-2-(4-chlomphényl)imidazo[1,2-*a*]pyridin-6-yl]phenyl}propan-2-ol ;
[2-Fluoro-6-(3-méthyl-2-phénylimidazo[1,2-*a*]pyridin-6-yl)phényl]méthanol ;
{2-[2-(4-chlorophényl)-3-méthylimidazo[1.2-*a*]pyridin-6-yl]-6-fluorophényl}méthanol ;
{2-[2-(3-Chlorophényl)-3-méthylimidazo[1,2-*a*]pyridin-6-yl]-6-fluomphényl}méthanol ;
{2-[2-(2,4-Difluorophényl)-3-méthylimidazo[1,2-*a*]pyridin-6-yl]-6-fluorophényl}méthanol ;
3-Chloro-6-(3-méthoxyméthylphényl)-2-(4-chlorophényl)imidazo[1,2-*a*]pyridine ;
{3-[3-Chloro-2-(4-chlorophényl)imidazo[1,2-*a*]pyridin-6-yl]-2,6-difluorophényl}méthanol.

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel phermeceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (I) selon l'une quelconque des revendications 1 à 4 pour la préparation d'un médicament destiné au traitement et à la prévention de Fostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies, de la sclérose en planque.

13. Utilisation d'un composé de formule (I) selon l'une quelconque des revendications 1 à 4 pour la préparation d'un médicament destiné au traitement et à la prévention de la schizophrénie, la dépression, la dépendance à une substance, les troubles du déficit de l'attention et de l'hyperactivité.

14. Procédé de synthèse des composés de formule (I) selon la revendication 1, carctérisé en ce que l'on fait réagir un composé de formule générale (IV), 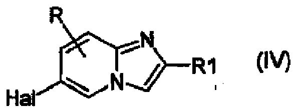 dans laquelle R et R1 sont définis selon la revendication 1 et Hal représente un atome d'halogène, avec un composé de formule générale (VII), 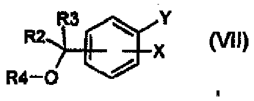 dans laquelle R2, R3, R4 et X sont définis selon la revendication 1 et Y représente un dérivé de bore ou d'étain.

15. Procédé de synthèse des composés de formule (I) selon la revendication 1 dans laquelle R est en position 3 du noyau imidazopyridine et R représente un groupe alkyle, 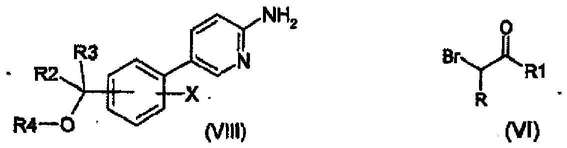 par condensation d'une aminopyridine de formule générale (VIII), dans laquelle R2, R3 et R4 et X sont définis selon la revendication 1, R4 étant différent d'un atome d'hydrogène, avec une bromocétone de formule générale (VI), dans laquelle R et R1 sont définis selon la revendication 1.

16. Procédé de synthèse des composés de formule (I) selon la revendication 1 dans laquelle R est en position 3 du noyau imidazopyridine et R4 représente un atome d'hydrogène, 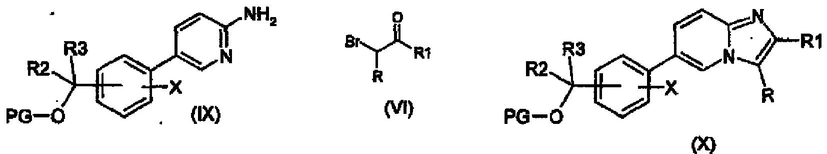 **caractérisé en ce qu'** une aminopyridine de formule générale (IX), dans laquelle R2, R3 et X sont définis selon la revendication 1, et PG représente un groupement protecteur de fonction hydroxyle, réagit avec une bromocétone de formule générale (VI), dans laquelle R1 défini selon la revendication 1, et R représente un groupe alkyle, pour obtenir des composés de formule générale (X) ;
le composé de formule générale (X) est ensuite soumis à une réaction de deprotection.

17. Procédé de synthèse des composés de formule générale (I) dans laquelle R est en position 3 du noyau imidazopyridine et représente un atome d'halogène, **caractérisé en ce que** l'on fait ragir un composé de formule générale (XII) 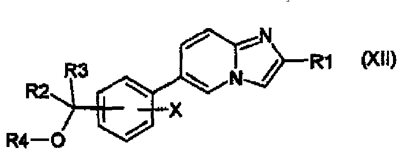 dans laquelle X, R1, R2, R3, R4 sont définis selon la revendication 1, avec un agent d'halogénation.

18. Procédé de synthèse des composés de formule générale (I) dans laquelle R est en position 3 du noyau imidazopyridine et représente un atome d'halogène, **caractérisé en ce que** l'on fait ragir un composé de formule générale (IV'), 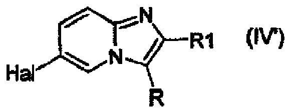 dans laquelle R1 est défini selon la revendication 1, R représente un atome de fluor ou de chlore et Hal représente un atome de brome ou d'iode, avec un composé de formule générale (VII), 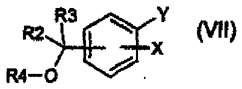 dans laquelle R2, R3, R4 et X sont définis selon la revendication 1 et Y représente un dérivé de bore ou d'étain.

19. Composés intermédiaires

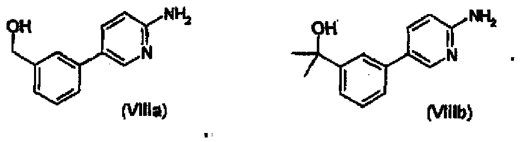

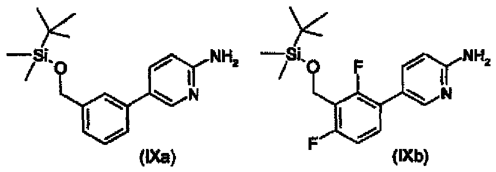

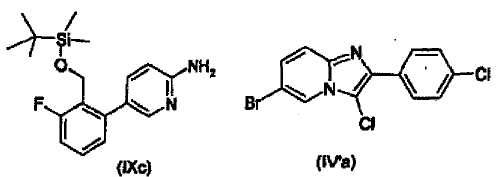

20. Procédé de synthèse selon la revendication 15, 16 ou 18, dans laquelle les composés de formules générales (VII), (IX) et (IV') sont les composés de formules (VIIIa), (VIIIb), (IXa), (IXb), (IXc) et (IV').

## Patentansprüche

1. Verbindungen der Formel (I): 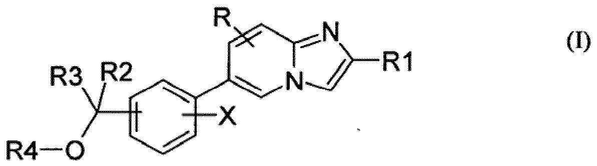 worin:
R₁ für eine Phenylgruppe oder eine Naphthylgruppe steht, wobei diese beiden Gruppen gegebenenfalls mit einem (einer) oder mehreren Atomen oder Gruppen substituiert sein können, die unabhängig voneinander ausgewählt sind aus den folgenden Atomen oder Gruppen: Halogen, (C₁-C₁₀)Alkyl, (C₁-C₁₀)Alkoxy, Halo(C₁-C₁₀)alkoxy;
X für 1 bis 2 Substituenten steht, gleich oder verschieden voneinander, ausgewählt aus Wasserstoff, Halogen;
R für 1 bis 2 Substituenten an Position 3, 7 oder 8 von Imidazo[1,2-α]pyridin steht, gleich oder verschieden voneinander, ausgewählt aus einem Halogen, (C₁-C₁₀)Alkyl;
R₂ und R₃ unabhängig voneinander für Folgendes stehen:
ein Wasserstoffatom,
eine (C₁-C₁₀)Alkylgruppe,
R₄ für Folgendes steht:
ein Wasserstoffatom,
eine (C₁-C₁₀)Alkylgruppe,
in Form einer Base oder eines Säureadditionssalzes.

2. Verbindungen der Formel (I) nach Anspruch 1, **dadurch gekennzeichnet, dass**:
sich die Gruppe 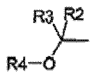 an Position 2, 3 oder 4 auf dem Phenylring, der sie trägt, befindet.

3. Verbindungen der Formel (I) nach Anspruch 1 oder 2, **dadurch gekennzeichnet, dass**:
R₁ für eine Phenylgruppe oder eine Naphthylgruppe steht, wobei diese beiden Gruppen gegebenenfalls mit einem (einer) oder mehreren Atomen oder Gruppen substituiert sein können, die unabhängig voneinander ausgewählt sind aus den folgenden Atomen oder Gruppen: Halogen, (C₁-C₁₀)Alkyl, (C₁-C₁₀)Alkoxy, Halo(C₁-C₁₀) alkoxy;
X für ein Wasserstoffatom steht,
R sich an Position 3, 7 oder 8 des Imidazo[1,2-α]pyridins befindet und für ein (C₁-C₁₀)Alkyl steht;
R₂ und R₃ unabhängig voneinander für ein Wasserstoffatom stehen;
R₄ für ein Wasserstoffatom steht,
in Form einer Base oder eines Säureadditionssalzes.

4. Verbindungen
{3-[2-(4-Chlorphenyl)-8-methylimidazo[1,2-α]pyridin-6-yl]phenyl}methanol;
{3-[2-(4-Chlorphenyl)-7-methylimidazo[1,2-α]pyridin-6-yl]phenyl}methanol;
[3-((3-Methyl-2-phenylimidazo[1,2-α]pyridin-6-yl)phenyl]methanol;
{3-[2-(2,4-Difluorphenyl)-3-methylimidazo[1,2-α]pyridin-6-yl]phenyl}methanol und sein Hydrochlorid;
{3-[2-(4-Chlor-3-methylphenyl)-3-methylimidazo[1,2-α]pyridin-6-yl]phenyl}methanol und sein Hydrochlorid;
{3-[2-(4-Chlorphenyl)-3-methylimidazo[1,2-α]pyridin-6-yl]phenyl}methanol und sein Hydrochlorid;
{3-[2-(4-Methoxyphenyl)-3-methylimidazo[1,2-α]pyridin-6-yl]phenyl}methanol und sein Hydrochlorid;
{3-[2-[4-(Difluormethyloxy)phenyl]-3-methylimidazo[1,2-α]pyridin-6-yl]phenyl}methanol und sein Hydrochlorid;
[3-((8-Methyl-2-naphthyl-2-ylimidazo[1,2-α]pyridin-6-yl)phenyl]methanol;
[4-((8-Methyl-2-naphthyl-2-ylimidazo[1,2-α]pyridin-6-yl)phenyl]methanol;
2-[3-(3-Methyl-2-phenylimidazo[1,2-α]pyridin-6-yl)phenyl]propan-2-ol;
2-{3-[2-(4-Chlorphenyl)-3-methylimidazo[1,2-α]pyridin-6-yl]phenyl}propan-2-ol;
2-{3-[2-(3-Chlorphenyl)-3-methylimidazo[1,2-α]pyridin-6-yl]phenyl}propan-2-ol;
2-{3-[2-(2,4-Difluorphenyl)-3-methylimidazo[1,2-α]pyridin-6-yl]phenyl}propan-2-ol;
2-{3-[2-(4-Methoxyphenyl)-3-methylimidazo[1,2-α]pyridin-6-yl]phenyl}propan-2-ol;
{2-Fluor-6-[2-(4-methoxyphenyl)-3-methylimidazo[1,2-α]pyridin-6-yl]phenyl}methanol;
[2,6-Difluor-3-(3-methyl-2-phenylimidazo[1,2-α]pyridin-6-yl)phenyl]methanol;
{3-[2-(4-Chlorphenyl)-3-methylimidazo[1,2-α]pyridin-6-yl]-2,6-difluorphenyl}methanol;
{3-[2-(3-Chlorphenyl)-3-methylimidazo[1,2-α]pyridin-6-yl]-2,6-difluorphenyl}methanol;
{2,6-Difluor-3-[2-(4-methoxyphenyl)-3-methylimidazo[1,2-α]pyridin-6-yl]-phenyl}methanol;
{3-[3-Chlor-2-(4-chlorphenyl)imidazo[1,2-α]pyridin-6-yl]phenyl}methanol;
2-{3-[2-(4-Chlorphenyl)-3-fluorimidazo[1,2-α]pyridin-6-yl]phenyl}propan-2-ol;
2-{3-[3-Chlor-2-(4-chlorphenyl)imidazo[1,2-α]pyridin-6-yl]phenyl}propan-2-ol;
[2-Fluor-6-(3-methyl-2-phenylimidazo[1,2-α]pyridin-6-yl)phenyl]methanol;
{2-[2-(4-Chlorphenyl)-3-methylimidazo[1,2-α]pyridin-6-yl]-6-fluorphenyl}methanol;
{2-[2-(3-Chlorphenyl)-3-methylimidazo[1,2-α]pyridin-6-yl]-6-fluorphenyl}methanol;
{2-[2-(2,4-Difluorphenyl)-3-methylimidazo[1,2-α]pyridin-6-yl]-6-fluorphenyl}methanol;
3-Chlor-6-(3-methoxymethylphenyl)-2-(4-chlorphenyl)imidazo[1,2-α]pyridin;
{3-[3-Chlor-2-(4-chlorphenyl)imidazo[1,2-α]pyridin-6-yl]-2,6-difluorphenyl}methanol.

5. Medikament, **dadurch gekennzeichnet, dass** es eine Verbindung der Formel (I) nach einem der Ansprüche 1 bis 4 oder ein Säureadditionssalz dieser Verbindung mit einer pharmazeutisch verträglichen Säure umfasst.

6. Pharmazeutische Zusammensetzung, **dadurch gekennzeichnet, dass** sie eine Verbindung der Formel (I) nach einem der Ansprüche 1 bis 4 oder ein pharmazeutisch verträgliches Salz dieser Verbindung sowie mindestens einen pharmazeutisch verträglichen Exzipienten umfasst.

7. Verwendung einer Verbindung der Formel (I) nach einem der Ansprüche 1 bis 4 zur Herstellung eines Medikaments, das zur Behandlung und Verhütung von neurodegenerativen Erkrankungen bestimmt ist.

8. Verwendung einer Verbindung der Formel (I) nach einem der Ansprüche 1 bis 4 zur Herstellung eines Medikaments, das zur Behandlung und Verhütung von Hirnverletzungen und Epilepsie bestimmt ist.

9. Verwendung einer Verbindung der Formel (I) nach einem der Ansprüche 1 bis 4 zur Herstellung eines Medikaments, das zur Behandlung und Verhütung von psychiatrischen Erkrankungen bestimmt ist.

10. Verwendung einer Verbindung der Formel (I) nach einem der Ansprüche 1 bis 4 zur Herstellung eines Medikaments, das zur Behandlung und Verhütung von entzündlichen Erkrankungen bestimmt ist.

11. Verwendung einer Verbindung der Formel (I) nach einem der Ansprüche 1 bis 4 zur Herstellung eines Medikaments, das zur Behandlung und Verhütung von Osteoporose und Krebsformen bestimmt ist.

12. Verwendung einer Verbindung der Formel (I) nach einem der Ansprüche 1 bis 4 zur Herstellung eines Medikaments, das zur Behandlung und Verhütung von Parkinson-Krankheit, Alzheimer-Krankheit, Tauopathien, multipler Sklerose bestimmt ist.

13. Verwendung einer Verbindung der Formel (I) nach einem der Ansprüche 1 bis 4 zur Herstellung eines Medikaments, das zur Behandlung und Verhütung von Schizophrenie, Depression, Substanzabhängigkeit, Aufmerksamkeitsdefizit/Hyperaktivitätsstörung bestimmt ist.

14. Verfahren zur Synthese der Verbindungen der Formel (I) nach Anspruch 1, **dadurch gekennzeichnet, dass** eine Verbindung der allgemeinen Formel (IV): 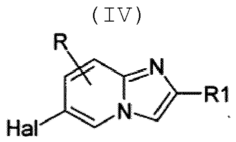 worin R und R1 nach Anspruch 1 definiert sind, und Hal für ein Halogenatom steht, mit einer Verbindung der allgemeinen Formel (VII): 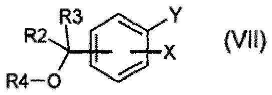 worin R2, R3, R4 und X nach Anspruch 1 definiert sind, und Y für ein Bor- oder Zinnderivat steht, umgesetzt wird.

15. Verfahren zur Synthese der Verbindungen der Formel (I) nach Anspruch 1, worin sich R an Position 3 des Imidazopyridinrings befindet und R für eine Alkylgruppe steht, 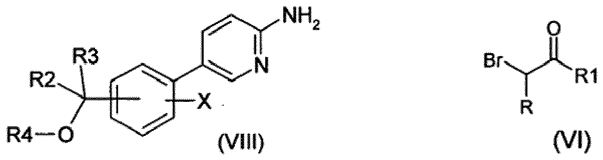 durch Kondensation eines Aminopyridins der allgemeinen Formel (VIII), worin R2, R3 und R4 und X nach Anspruch 1 definiert sind, R4 verschieden von einem Wasserstoffatom ist, mit einem Bromketon der allgemeinen Formel (VI), worin R und R1 nach Anspruch 1 definiert sind.

16. Verfahren zur Synthese der Verbindungen der Formel (I) nach Anspruch 1, worin sich R an Position 3 des Imidazopyridinrings befindet und R4 für ein Wasserstoffatom steht, 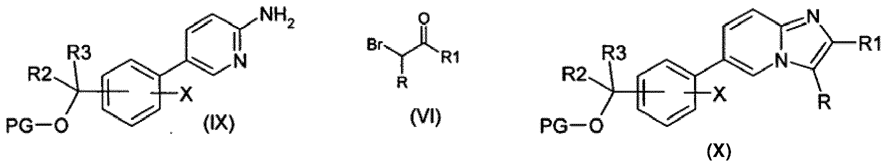 **dadurch gekennzeichnet, dass** ein Aminopyridin der allgemeinen Formel (IX), worin R2, R3 und X nach Anspruch 1 definiert sind und PG für eine Schutzgruppe der Hydroxylfunktion steht, mit einem Bromketon der allgemeinen Formel (VI), worin R1 nach Anspruch 1 definiert ist und R für eine Alkylgruppe steht, umgesetzt wird, um Verbindungen der allgemeinen Formel (X) zu erhalten;
wobei die Verbindung der allgemeinen Formel (X) anschließend einer Entschützungsreaktion unterzogen wird.

17. Verfahren zur Synthese der Verbindungen der allgemeinen Formel (I), worin sich R an Position 3 des Imidazopyridinrings befindet und für ein Halogenatom steht, **dadurch gekennzeichnet, dass** eine Verbindung der allgemeinen Formel (XII) : 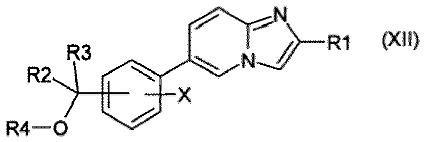 worin X, R1, R2, R3, R4 nach Anspruch 1 definiert sind, mit einem Halogenierungsmittel umgesetzt wird.

18. Verfahren zur Synthese der Verbindungen der allgemeinen Formel (I), worin sich R an Position 3 des Imidazopyridinrings befindet und für ein Halogenatom steht, **dadurch gekennzeichnet, dass** eine Verbindung der allgemeinen Formel (IV'): 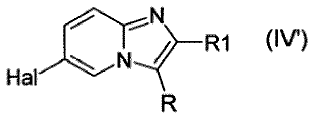 worin R1 nach Anspruch 1 definiert ist, wobei R für ein Fluor- oder Chloratom steht und Hal für ein Brom- oder Iodatom steht, mit einer Verbindung der allgemeinen Formel (VII): 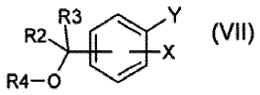 worin R2, R3, R4 und X nach Anspruch 1 definiert sind, und Y für ein Bor- oder Zinnderivat steht, umgesetzt wird.

19. Zwischenverbindungen:

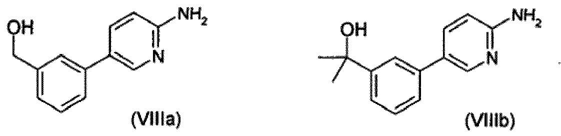

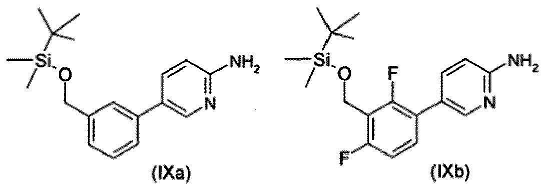

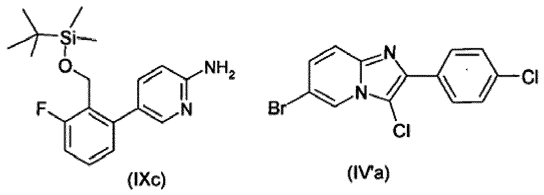

20. Verfahren zur Synthese nach Anspruch 15, 16 oder 18, worin die Verbindungen der allgemeinen Formeln (VIII), (IX) und (IV') die Verbindungen der Formeln (VIIIa), (VIIIb), (IXa), (IXb), (IXc) und (IV') sind.

## Claims

1. Compound of formula (I): 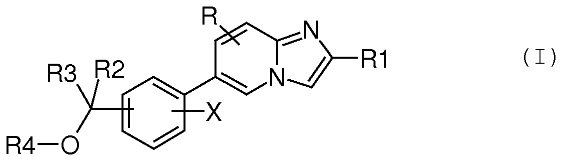 in which:
R₁ represents a phenyl group or a naphthyl group, it being possible for these two groups to be optionally substituted with one or more atoms or groups chosen,
independently of one another, from the following atoms or groups: halogen, (C₁-C₁₀) alkyl, (C₁-C₁₀) alkoxy or
halo (C₁-C₁₀) alkoxy;
X represents from 1 to 2 substituents, which are identical to or different from one another, chosen from hydrogen or halogen;
R represents, at position 3, 7 or 8 of the imidazo[1,2-*a*]pyridine, from 1 to 2 substituents, which are identical to or different from one another, chosen from a halogen or (C₁-C₁₀)alkyl;
R₂ and R₃ represent, independently of one another,
a hydrogen atom;
a (C₁-C₁₀)alkyl group;
R₄ represents:
a hydrogen atom;
a (C₁-C₁₀)alkyl group;
in the form of a base or of an addition salt with an acid.

2. Compounds of formula (I) according to Claim 1, **characterized in that**: the group 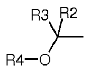 is at position 2, 3 or 4 on the phenyl nucleus which bears it.

3. Compounds of formula (I) according to Claim 1 or 2, **characterized in that**:
R₁ represents a phenyl group or a naphthyl group, it being possible for these two groups to be optionally substituted with one or more atoms or groups chosen, independently of one another, from the following atoms or groups: halogen, (C₁-C₁₀) alkyl, (C₁-C₁₀) alkoxy or
halo (C₁-C₁₀) alkoxy;
X represents a hydrogen atom,
R is at position 3, 7 or 8 of the imidazo[1,2-*a*]pyridine and represents a (C₁-C₁₀)alkyl;
R₂ and R₃ represent, independently of one another, a hydrogen atom;
R₄ represents a hydrogen atom;
in the form of a base or of an addition salt with an acid.

4. Compounds
{3-[2-(4-chlorophenyl)-8-methylimidazo[1,2-*a*]pyridin-6-yl]phenyl}methanol;
{3-[2-(4-chlorophenyl)-7-methylimidazo[1,2-*a*]pyridin-6-yl]phenyl}methanol;
[3-((3-methyl-2-phenylimidazo[1,2-*a*]pyridin-6-yl)phenyl]methanol;
{3-[2-(2,4-difluorophenyl)-3-methylimidazo[1,2-*a*]pyridin-6-yl]phenyl}methanol and the hydrochloride thereof;
{3-[2-(4-chloro-3-methylphenyl)-3-methylimidazo[1,2-*a*]pyridin-6-yl]phenyl}methanol and the hydrochloride thereof;
{3-[2-(4-chlorophenyl)-3-methylimidazo[1,2-*a*]pyridin-6-yl]phenyl}methanol and the hydrochloride thereof;
{3-[2-(4-methoxyphenyl)-3-methylimidazo[1,2-*a*]pyridin-6-yl]phenyl}methanol and the hydrochloride thereof;
{3-[2-[4-(difluoromethyloxy)phenyl]-3-methylimidazo[1,2-*a*]pyridin-6-yllphenyllmethanol and the hydrochloride thereof;
[3-((8-methyl-2-naphthyl-2-ylimidazo[1,2-*a*]pyridin-6-yl)phenyl]methanol;
[4-((8-methyl-2-naphthyl-2-ylimidazo[1,2-*a*]pyridin-6-yl)phenyl]methanol;
2-[3-(3-methyl-2-phenylimidazo[1,2-*a*]pyridin-6-yl)phenyl]propan-2-ol;
2-{3-[2-(4-chlorophenyl)-3-methylimidazo[1,2-*a*]pyridin-6-yl]phenyl}propan-2-ol;
2-{3-[2-(3-chlorophenyl)-3-methylimidazo[1,2-*a*]pyridin-6-yl]phenyl}propan-2-ol;
2-{3-[2-(2,4-difluorophenyl)-3-methylimidazo[1,2-*a*]pyridin-6-yl]phenyl}propan-2-ol;
2-{3-[2-(4-methoxyphenyl)-3-methylimidazo[1,2-*a*]pyridin-6-yl]phenyl}propan-2-ol;
{2-fluoro-6-[2-(4-methoxyphenyl)-3-methylimidazo[1,2-*a*]pyridin-6-yl]phenyl}methanol;
[2,6-difluoro-3-(3-methyl-2-phenylimidazo[1,2-*a*]pyridin-6-yl)phenyl]methanol;
{3-[2-(4-chlorophenyl)-3-methylimidazo[1,2-*a*]pyridin-6-yl]-2,6-difluorophenyl}methanol;
{3-[2-(3-chlorophenyl)-3-methylimidazo[1,2-*a*]pyridin-6-yl]-2,6-difluorophenyllmethanol;
{2,6-difluoro-3-[2-(4-methoxyphenyl)-3-methylimidazo[1,2-*a*]pyridin-6-yl]-phenyl}methanol;
{3-[3-chloro-2-(4-chlorophenyl)imidazo[1,2-*a*]pyridin-6-yl]phenyl}methanol;
2-{3-[2-(4-chlorophenyl)-3-fluoroimidazo[1,2-*a*]pyridin-6-yl]phenyl}propan-2-ol;
2-{3-[3-chloro-2-(4-chlorophenyl)imidazo[1,2-*a*]pyridin-6-yl]phenyl}propan-2-ol;
[2-fluoro-6-(3-methyl-2-phenylimidazo[1,2-*a*]pyridin-6-yl)phenyl]methanol;
{2-[2-(4-chlorophenyl)-3-methylimidazo[1,2-*a*]pyridin-6-yl]-6-fluorophenyl}methanol;
{2-[2-(3-chlorophenyl)-3-methylimidazo[1,2-*a*]pyridin-6-yl]-6-fluorophenyl}methanol;
{2-[2-(2,4-difluorophenyl)-3-methylimidazo[1,2-*a*]pyridin-6-yl]-6-fluorophenyl}methanol;
3-chloro-6-(3-methoxymethylphenyl)-2-(4-chlorophenyl)imidazo[1,2-*a*]pyridine;
{3-[3-chloro-2-(4-chlorophenyl)imidazo[1,2-*a*]pyridin-6-yl]-2,6-difluorophenyl}methanol.

5. Medicament, **characterized in that** it comprises a compound of formula (I) according to any one of Claims 1 to 4, or an addition salt of this compound with a pharmaceutically acceptable acid.

6. Pharmaceutical composition, **characterized in that** it comprises a compound of formula (I) according to any one of Claims 1 to 4, or a pharmaceutically acceptable salt of this compound, and also at least one pharmaceutically acceptable excipient.

7. Use of a compound of formula (I) according to any one of Claims 1 to 4, for the preparation of a medicament for use in the treatment and prevention of neurodegenerative diseases.

8. Use of a compound of formula (I) according to any one of Claims 1 to 4, for the preparation of a medicament for use in the treatment and prevention of cerebral traumas and of epilepsy.

9. Use of a compound of formula (I) according to any one of Claims 1 to 4, for the preparation of a medicament for use in the treatment and prevention of psychiatric diseases.

10. Use of a compound of formula (I) according to any one of Claims 1 to 4, for the preparation of a medicament for use in the treatment and prevention of inflammatory diseases.

11. Use of a compound of formula (I) according to any one of Claims 1 to 4, for the preparation of a medicament for use in the treatment and prevention of osteoporosis and cancers.

12. Use of a compound of formula (I) according to any one of Claims 1 to 4, for the preparation of a medicament for use in the treatment and prevention of Parkinson's disease, Alzheimer's disease, tauopathies and multiple sclerosis.

13. Use of a compound of formula (I) according to any one of Claims 1 to 4, for the preparation of a medicament for use in the treatment and prevention of schizophrenia, depression, substance dependence and attention deficit hyperactivity disorders.

14. Process for synthesizing the compounds of formula (I) according to Claim 1, **characterized in that** a compound of general formula (IV) 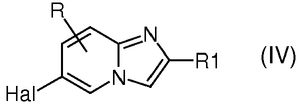 in which R and R1 are defined according to Claim 1 and Hal represents a halogen atom, is reacted with a compound of general formula (VII) 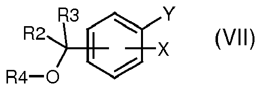 in which R2, R3, R4 and X are defined according to Claim 1 and Y represents a boron or tin derivative.

15. Process for synthesizing the compounds of formula (I) according to Claim 1, in which R is at position 3 of the imidazopyridine nucleus and R represents an alkyl group 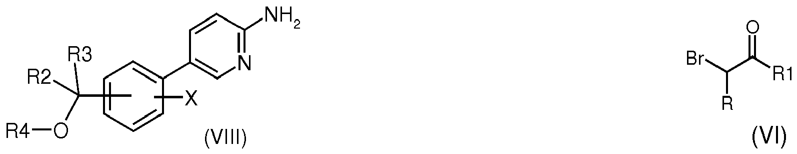 by condensation of an aminopyridine of general formula (VIII), in which R2, R3, R4 and X are defined according to Claim 1, R4 being different from a hydrogen atom, with a bromoketone of general formula (VI), in which R and R1 are defined according to Claim 1.

16. Process for synthesizing the compounds of formula (I) according to Claim 1, in which R is at position 3 of the imidazopyridine nucleus and R4 represents a hydrogen atom, 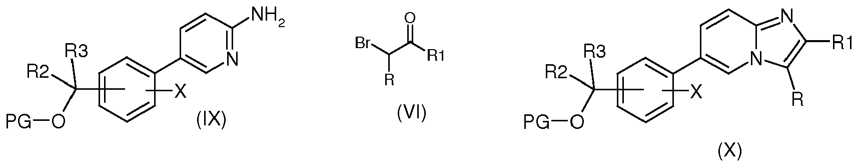 **characterized in that** an aminopyridine of general formula (IX), in which R2, R3 and X are defined according to Claim 1, and PG represents a hydroxyl-function-protecting group, reacts with a bromoketone of general formula (VI), in which R1 is defined according to Claim 1 and R represents an alkyl group, so as to obtain compounds of general formula (X);
the compound of general formula (X) is then subjected to a deprotection reaction.

17. Process for synthesizing the compounds of general formula (I) in which R is at position 3 of the imidazopyridine nucleus and represents a halogen atom, **characterized in that** a compound of general formula (XII) 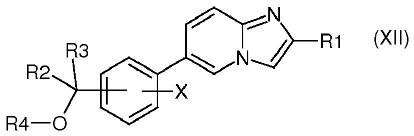 in which X, R1, R2, R3 and R4 are defined according to Claim 1, is reacted with a halogenating agent.

18. Process for synthesizing the compounds of general formula (I) in which R is at position 3 of the imidazopyridine nucleus and represents a halogen atom, **characterized in that** a compound of general formula (IV') 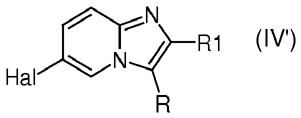 in which R1 is defined according to Claim 1, R represents a fluorine or chlorine atom and Hal represents a bromine or iodine atom, is reacted with a compound of general formula (VII) 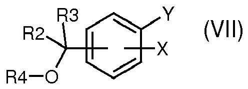 in which R2, R3, R4 and X are defined according to Claim 1 and Y represents a boron or tin derivative.

19. Intermediate compounds

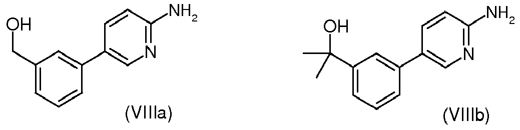

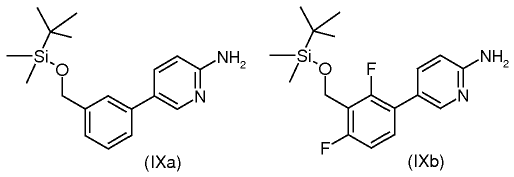

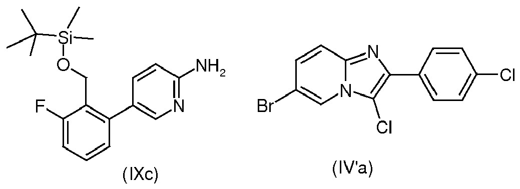

20. Synthesis process according to Claim 15, 16 or 18, in which the compounds of general formulae (VIII), (IX) and (IV') are the compounds of formulae (VIIIa), (VIIIb), (IXa), (IXb), (IXc) and (IV').
